# EUROPÄISCHE PATENTSCHRIFT

(11) **EP 1 786 913 B1**
(45) Veröffentlichungstag und Bekanntmachung des Hinweises auf die Patenterteilung: **17.08.2011**
(21) Anmeldenummer: 05782434.4
(22) Anmeldetag: 23.08.2005
(51) Int. Cl.: C12P 7/62

(54) **VERFAHREN ZUR HERSTELLUNG VON DIARYLCYCLOALKYLDERIVATEN**
METHOD FOR THE PRODUCTION OF DIARYLCYCLOALKYL DERIVATIVES
PROCEDE DE PRODUCTION DE DERIVES DE DIARYLCYCLOALKYLE

(30) Priorität: 23.08.2004 DE 102004040736
(43) Veröffentlichungstag der Anmeldung: 23.05.2007
(73) Patentinhaber: Sanofi-Aventis Deutschland GmbH, 65929 Frankfurt am Main (DE)
(72) Erfinder: SALAGNAD, Christophe, 65929 Frankfurt am Main (DE); ZOCHER, Frank, 65929 Frankfurt am Main (DE); BURGARD, Andreas, 65929 Frankfurt am Main (DE); JUNKER, Bernd, 65929 Frankfurt am Main (DE); HOERLEIN, Rolf, 65929 Frankfurt am Main (DE); STUEDEMANN, Thomas, 65929 Frankfurt am Main (DE); MAIER, Claus-Jürgen, 65926 Frankfurt am Main (DE); HACHTEL, Jochen, 65929 Frankfurt am Main (DE); HOLLA, Wolfgang, 65929 Frankfurt am Main (DE); TAPPERTZHOFEN, Christoph, 60385 Frankfurt (DE); KULITZSCHER, Berndt, 65929 Frankfurt am Main (DE); MUTTI, Stéphane, F-94170 Le Perreux sur Marne (FR)
(86) Internationale Anmeldenummer: PCT/EP2005/009095
(87) Internationale Veröffentlichungsnummer: WO 2006/021420

(56) Entgegenhaltungen:
- WO-A-03/020269
- WO-A-2004/075815
- WO-A-2004/076390
- WO-A-2005/040394
- NICOLOSI G ET AL: "Desymmetrization of cis-1,2-Dihydroxycycloalkanes by Stereoselective Lipase Mediated Esterification" TETRAHEDRON: ASYMMETRY, ELSEVIER SCIENCE PUBLISHERS, AMSTERDAM, NL, Bd. 6, Nr. 2, Februar 1995 (1995-02), Seiten 519-524, XP004048459 ISSN: 0957-4166
- BANNARD, ROBERT A. B. ET AL: "Utility of the dehalogenation-deetherification sequence for the proof of structure of methoxyhalocyclohexanols and methoxyhalocyclopentanols. Synthesis of the cis- and trans-2 and 3-methoxycyclohexanols and -cyclopentanols" CANADIAN JOURNAL OF CHEMISTRY , 45(21), 2605-11 CODEN: CJCHAG; ISSN: 0008-4042, 1967, XP009065785
- HARADA T ET AL: "Enantiodifferentiating functionalization of cis-cycloalkane-1,2-diols and cis-endo-5-norbornen-2,3-ylenebis(methanol ) via chiral spiroacetals derived from 1-menthone" JOURNAL OF ORGANIC CHEMISTRY, AMERICAN CHEMICAL SOCIETY. EASTON, US, Bd. 54, Nr. 11, Mai 1989 (1989-05), Seiten 2599-2605, XP002973762 ISSN: 0022-3263
- GATTI, ROBERTO G. P. ET AL: "Palladium-catalyzed enantiodivergent synthesis of cis- and trans-4-aminocyclohex-2-enols" JOURNAL OF THE CHEMICAL SOCIETY, PERKIN TRANSACTIONS 1: ORGANIC AND BIO-ORGANIC CHEMISTRY , (4), 577-584 CODEN: JCPRB4; ISSN: 0300-922X, 1997, XP002378858
- NARA, M. ET AL: "Stereochemical studies. LVII. Synthesis of optically active compounds by the novel use of meso-compounds. 1. Efficient synthesis of two structural types of optically pure prostaglandin intermediates" TETRAHEDRON , 36(22), 3161-70 CODEN: TETRAB; ISSN: 0040-4020, 1980, XP002378859
- CURRAN T T ET AL: "The Preparation of Optically Active 2-Cyclopenten-1,4-Diol Derivatives from Furfuryl Alcohol" TETRAHEDRON, ELSEVIER SCIENCE PUBLISHERS, AMSTERDAM, NL, Bd. 53, Nr. 6, 10. Februar 1997 (1997-02-10), Seiten 1983-2004, XP004105280 ISSN: 0040-4020
- THEIL F ET AL: "INVESTIGATION OF THE PANCREATIN-CATALYZED ACYLATION OF CIS-CYCLOPENT-2-ENE-1,4-DIOL WITH VARIOUS TRICHLOROETHYL AND VINYL ALKANOATES" LIEBIGS ANNALEN DER CHEMIE, VERLAG CHEMIE GMBH. WEINHEIM, DE, Nr. 3, 1991, Seiten 195-200, XP008031881 ISSN: 0170-2041
- BERNASCONI, C. ET AL: "Stereoselective photolysis of 2-(.alpha.-tetrahydropyranyloxy)-3- tetrahydropyrones" JOURNAL OF HETEROCYCLIC CHEMISTRY , 17(1), 45-8 CODEN: JHTCAD; ISSN: 0022-152X, 1980, XP002378860
- JOHNSON C R ET AL: "Enzymatic asymmetrization of meso-2-cycloalken-1,4-diols and their diacetates in organic and aqueous media" TETRAHEDRON LETTERS, ELSEVIER, AMSTERDAM, NL, Bd. 33, Nr. 48, 1992, Seiten 7287-7290, XP002027733 ISSN: 0040-4039
- PATTI, ANGELA ET AL: "Enantioselective Synthesis of (-)- and (+)-Conduritol F via Enzymic Asymmetrization of cis-Cyclohexa-3,5-diene-1,2-diol" JOURNAL OF ORGANIC CHEMISTRY, 61(18), 6458-6461 CODEN: JOCEAH; ISSN: 0022-3263, 1996, XP002398389
- MATTSON A ET AL: "KINETIC RESOLUTION OF CHIRAL AUXILIARIES WITH C2-SYMMETRY BY LIPASE-CATALYZED ALCOHOLYSIS AND AMINOYLSIS" ACTA CHEMICA SCANDINAVICA, MUNKSGAARD, COPENHAGEN, DK, Bd. 50, Nr. 10, 1996, Seiten 918-921, XP009071804 ISSN: 0904-213X

## Beschreibung

### Verfahren zur Herstellung von DiarylCycloalkylderivaten

Die Erfindung betrifft ein Verfahren zur Herstellung von Diarylcycloalkylderivaten der allgemeinen Formel (I). Weiterhin betrifft die vorliegende Erfindung neue Zwischenprodukte, die beim erfindungsgemäßen Verfahren gebildet werden.

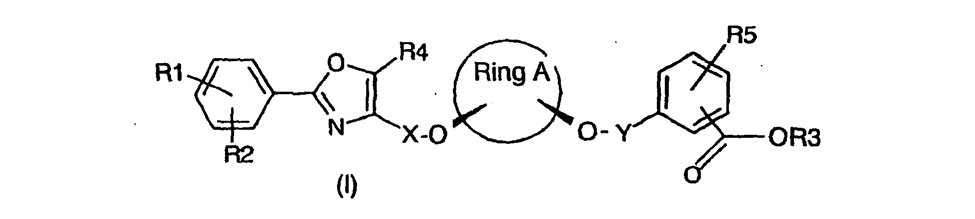

Die Verbindungen der Formel (I) sind Aktivatoren für Peroxisom-Proliferator aktivierte Rezeptoren (PPAR-Aktivator) und sind bereits aus WO 03/020269 bekannt. Von den in WO 03/020269 beschriebenen PPAR-Aktivatoren haben sich diejenigen als wirkungsvolle PPAR-Aktivatoren erwiesen, die eine cis-Substitution des X- und Y-enthaltenden Substituenten am zentralen Ring A aufweisen. Dies trifft insbesondere auf Verbindungen zu, bei denen der Ring A = Cyclohexyl ist, vorzugsweise cis-1,3-Cyclohexyl.

Bei der Synthese beziehungsweise Isolierung der gewünschten Zielmolekül gemäß Formel (I) bereiten hauptsächlich zwei Faktoren Schwierigkeiten. Zum einen ist dies die cis/trans-Isomerie der Substituenten des Ringes A. Da bei den Verbindungen der Formel (I) die entsprechenden cis-Isomere wirkungsvollere PPAR-Aktivatoren sind als die entsprechenden trans-Isomere, ist es ratsam, bereits zu Beginn der Synthese in den entsprechenden Zwischenstufen die jeweiligen trans-Isomere des Ringes A abzutrennen, um unnötige Ausbeuteverluste zu vermeiden. Zum anderen muss auch bei alleiniger Betrachtung des entsprechenden cis-Isomere des Ringes A berücksichtigt werden, dass bei den meisten Zwischenprodukten sowie beim Zielmolekül der Formel (I) zwei chirale Kohlenstoffatome vorhanden sind und der Ring A mit zwei unterschiedlichen Resten (X, Y) substituiert ist. Folglich muss bei der Verknüpfung des Ring A mit beispielsweise dem X-enthaltenden Substituenten berücksichtigt werden, dass sich bei einer äquimolaren Umsetzung ein racemisches Gemisch ausbildet, weil dieser Substituent prinzipiell mit beiden funktionellen Gruppen des Ringes A verknüpft werden kann. Wird dies nicht beachtet, liegen auch die Verbindungen der Formel (I) als racemisches Gemisch vor.

Mit dem in WO 03/020269 beschriebenen Herstellungsverfahren für PPAR-Aktivatoren lassen sich zwar die Verbindungen der Formel (I) prinzipiell in enantiomerenreiner Form herstellen, allerdings weist das darin beschriebene Verfahren einige signifikante Nachteile auf: Einsatz und Entsorgung von giftigen Zinnverbindungen und Cäsiumfluorid; Einsatz und Entsorgung von Iodid-haltigen Verbindungen; Racemische Synthese, d.h. nach Abtrennung des nicht benötigten Enantiomers durch chirale Chromatographie fällt mindestens die Hälfte der teuren Ausgangsmaterialien als Abfall an, die chirale Chromatographie muss zusätzlich mit achiraler Chromatographie verknüpft werden; die Hälfte des Produktes bzw. der dafür eingesetzten wertvollen Ausgangsmaterialien gehen bei einer Racemattrennung in die beiden Enantiomere verloren; das "falsche" Enantiomer kann nicht rezyklisiert und muss als Abfall entsorgt werden; Einsatz von Natriumhydrid als Base und N,N-Dimethylformamid als Lösungsmittel (potentiell exotherme Zersetzung).

Um einen Enantiomerenüberschuß beziehungsweise eine enantiomerenreine Verbindung der Formel (I) herstellen zu können, ist nach dem in WO 03/020269 beschriebenen Verfahren eine chirale Chromatographie zwingend notwendig. Insbesondere im großtechnischen Maßstab erweisen sich die mit der chiralen Chromatographie verbundenen hohen Kosten als Hauptnachteil dieses Verfahrens.

Ein alternatives Verfahren zur Herstellung von PPAR-Aktivatoren, die unter die in WO 03/020269 beschriebenen PPAR-Aktivatoren fallen, wird in der internationalen Anmeldung mit der Nummer WO 2004/076390 beschrieben. In diesem Verfahren, das auf cis-1,3-disubstituierte Cyclohexan-Derivate beschränkt ist, wird zunächst cis-1,3-Cyclohexandiol entweder mit einer Schutzgruppe (Benzyl oder Silyl) oder bereits mit einem der beiden Substituenten des Zielmoleküls alkyliert, wobei sich das racemische Gemisch der entsprechenden monoalkylierten cis-Verbindung bildet. Dieses Zwischenprodukt wird wiederum entweder mit einem Acyldonor umgesetzt, worauf dieses ebenfalls als Racemat vorliegende monoalkylierte und monoacylierte Zwischenprodukt über eine enzymatische Esterspaltung sowie anschließender Chromatographie in zwei Fraktionen aufgetrennt wird, aus denen sich jeweils in getrennte Reaktionsführungen die beiden Enantiomere des Zielmoleküls synthetisieren lassen. Alternativ kann das racemische monoalkylierte Zwischenprodukt durch enzymatische Esterbildung sowie anschließender Chromatographie in zwei Fraktionen aufgetrennt werden, aus denen wiederum in zwei separaten Ansätzen die beiden enantiomeren Formen des Zielmoleküls synthetisiert werden können. Nachteilig bei diesem Verfahren ist insbesondere, dass trotz der Vermeidung von chiraler Chromatographie zunächst ein racemisches Zwischenprodukt gebildet wird, aus dem sich zwangsweise die beiden enantiomeren Formen des Zielmoleküls ergeben. Sofern die Synthesevariante über die zuerst eingeführte Schutzgruppe benutzt wird, müssen die benzylhaltigen Schutzgruppen durch Hydrierung abgetrennt werden. Bei dieser Hydrierung kann zu einem gewissen Grad auch der bereits an der entsprechenden Zwischenstufe angebundene erste Substituent des Zielmoleküls wieder entfernt werden, was zu einem Ausbeuteverlust führt. Silylhaltige Schutzgruppen werden mit Fluorid abgetrennt, auch dies führt zu weiteren Nebenreaktionen bei den übrigen Substituenten der entsprechenden Zwischenverbindungen und ist folglich zu vermeiden.

Die Verwendung von Enzymen zur Auftrennung von racemischen Gemischen diverser Verbindungen (Ausgangsmaterialien oder Zwischenstufen) ist in der Literatur schon mehrfach beschrieben worden. Das Auffinden geeigneter Enzyme zur enantioselektiven Trennung des jeweils aufzutrennenden racemischen Gemisches bereitet jedoch Schwierigkeiten.

So beschreiben T. Hirata et al., Chirality 9: 250-253 (1997) die Hydrolyse von *cis-*und *trans*-1,3-Diacetoxycyclohexan zu Acyloxycyclohexanolen in Gegenwart von kultivierten Pflanzenzellen von Brunnenlebermoos (*Marchantia polymorpha*). Hierzu ist die Kultivierung der Pflanzenzellen notwendig, die zugehörigen Enzyme sind nicht bekannt. Der Enantiomerenüberschuß bei der Hydrolyse von Meso-cis-1,3-Diacetoxycyclohexan beträgt hier nur 15 % für das (*1R*,*3S*)-1-Acetoxy-cyclohexan-3-ol. *trans*-1,3-Diacetoxycyclohexan wird zu (*1R*,*3R*)-3-Acetoxy-cyclohexan-1-ol (60 % Ausbeute) mit 27 % EnantiomerenüberschuB und Cyclohexan-1,3-diol (70 % Ausbeute) umgesetzt. Diese Methode eignet sich daher nicht zur Herstellung eines akzeptablen Enantiomerenüberschusses beziehungsweise von enantiomerenreinem *cis*-1S-Acytoxycyclohexan-3R-ol.

K. Laumen et al., J. Chem. Soc., Chem. Common., (1986) 1298 - 1299 beschrieben die enzymatische Hydrolyse von cis-1.4-Diacetoxycyclopent-2-en in Gegenwart von Lipasen wie Pseudomonas species oder Mucor miehei. Bei einem Umsatz von ca. 50 % wird ein monoacetyliertes Enantiomer mit einer Enantiomerenreinheit von 95 bis 97 % gebildet. Die Enantiomerenreinheit lässt sich durch Umkristallisation auf über 99 % erhöhen.

Die der vorliegenden Erfindung zugrundeliegende Aufgabe besteht darin, ein neues Verfahren zur Herstellung PPAR-Aktivatoren der allgemeinen Formel (I) bereitzustellen, das nicht die Nachteile der aus dem Stand der Technik bekannten Verfahren aufweist. Insbesondere soll ein Verfahren bereit gestellt werden, mit dem die PPAR-Aktivatoren der allgemeinen Formel (I) in einem geeigneten Enantiomerenüberschuss, d.h. in hoher Enantioselektivität, hergestellt werden können, ohne dass der Einsatz von chiraler Chromatographie erforderlich ist.

Die Aufgabe wird gelöst durch ein Verfahren zur Herstellung einer Verbindung der allgemeinen Formel (I), umfassend die folgenden Schritte, wobei:
a1) eine Verbindung der Formel (IX) mit Wasser zu einer Verbindng der Formel (V) in Gegenwart der Lipase B aus Candida antarctica
   , umgesetzt wird, oder
a2) eine Verbindung der Formel (X) mit mindestens einem Acyldonor zur Verbindung (V) in Gegenwart der Lipase B aus Candida antarctica
   , umgesetzt wird,
b) die Verbindung (V) in Gegenwart eines sauren Katalysators mit einer Verbindung, die die basenstabile und säurelabile Ketal-oder-ketal- Schutzgruppe Z3 ausbilden kann, zur Verbindung der Formel (VIII) umgesetzt wird und
c) die Verbindung (VIII) in Gegenwart eines Nukleophils zu einer Verbindung der Formel (II) umgesetzt wird,
d) die Verbindung (II) in Gegenwart einer Base B1 mit einer Verbindung der Formel (VI) zu einer Verbindung der Formel (IIIa) oder mit einer Verbindung
   der Formel (VII) zu einer Verbindung der Formel (IIIb) umgesetzt wird, 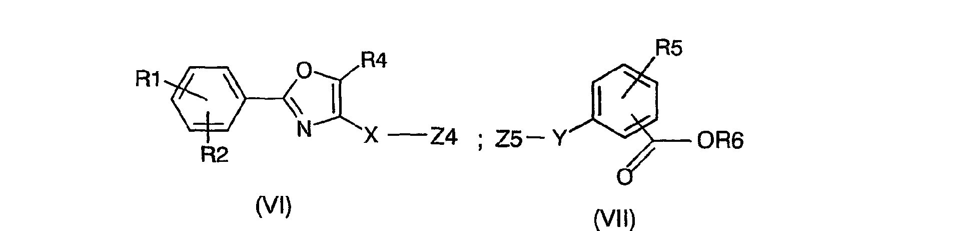
e) die Verbindung (IIIa) zu einer Verbindung der Formel (IVa) oder die Verbindung (IIIb) zu einer Verbindung der Formel (IVb) umgesetzt wird, wobei die jeweilige Umsetzung mit einem Alkohol in Gegenwart eines sauren Katalysators erfolgt,
f) die Verbindung (IVa) mit der Verbindung (VII) oder die Verbindung (IVb) mit der Verbindung (VI) zu einer Verbindung der Formel (Ia) in Gegenwart der Base B1 umgesetzt wird und
g) gegebenenfalls die Verbindung (Ia) zu der Verbindung (I) hydrolysiert oder
   hydrogenolysiert wird, sofern R3 gleich H ist,
wobei die Verbindungen (IX) und (X) jeweils als reines cis-Isomer oder als cis/trans Gemische vorliegen,
und worin die Variablen und Substituenten jeweils die nachfolgende Bedeutung haben:
Ring A ist C₃-C₈-Cycloalkyl oder C₃-C₈-Cycloalkenyl, wobei in den Cycolalkyl-oder Cycloalkenylringen ein oder mehrere Kohlenstoffatome durch Sauerstoffatome ersetzt sein können,
R1, R2, R4 und R5 sind unabhängig voneinander H, F, Cl, Br, OH, NO₂, CF₃, OCF₃, C₁-C₆-Alkyl oder -O-(C₁-C₆-Alkyl),
R3 ist H, C₁-C₆-Alkyl oder Benzyl, die gegebenenfalls mit F, Cl, Br, OH, NO₂, CF₃, OCF₃, C₁-C₆Alkyl oder -O-(C₁-C₆-Alkyl) substituiert sein können,
R6 ist C₁-C₆-Alkyl oder Benzyl, die gegebenenfalls mit F, Cl, Br, OH, NO_{2,} CF₃, OCF₃, C₁-C₆-Alkyl oder -O-(C₁-C₆-Alkyl) substituiert sein können,
X ist C₁-C₆-Alkyl, wobei in der Alkylgruppe ein oder mehrere Kohlenstoffatome durch Sauerstoffatome ersetzt sein können,
Y ist C₁-C₆-Alkyl, wobei in der Alkylgruppe ein oder mehrere Kohlenstoffatome durch Sauerstoffatome ersetzt sein können,
Z1 und Z2 sind unabhängig voneinander eine säurestabile Schutzgruppe, aus der Reihe -C(O)-C₁-C₆-Alkyl oder -C(O)-Phenyl,
Z3 ist eine basenstabile und säurelabile Acetal-oder Ketal Schutzgruppe
Z4 und Z5 sind unabhängig voneinander eine Abgangsgruppe,
B1 ist ein tertiäres Erdalkalialkoholat, tertiäres Alkalialkoholat, Erdalkaliamid, Alkaliamid, Erdalkalisilazid, Alkalisilazid oder Alkalihydrid.

Die in den vorstehenden Verfahrensschritten erwähnten. Verbindungen ergeben sich aus dem nachfolgenden Schema I, das zur Verdeutlichung des erfindungsgemäßen Verfahrens dient.

### Schema I

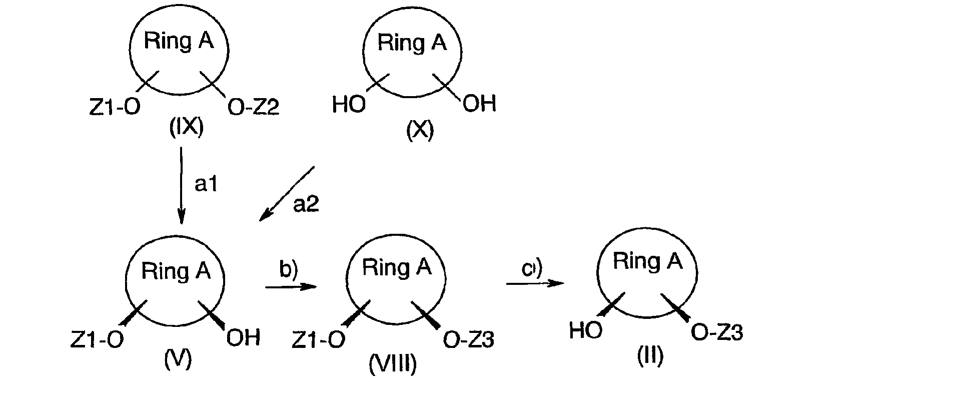

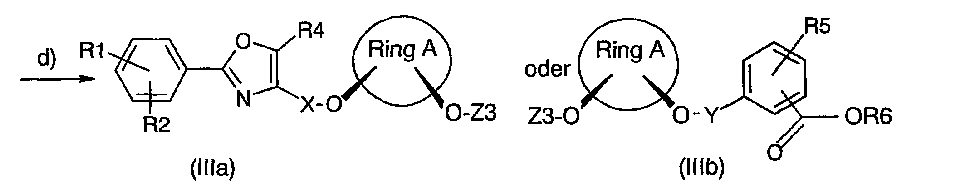

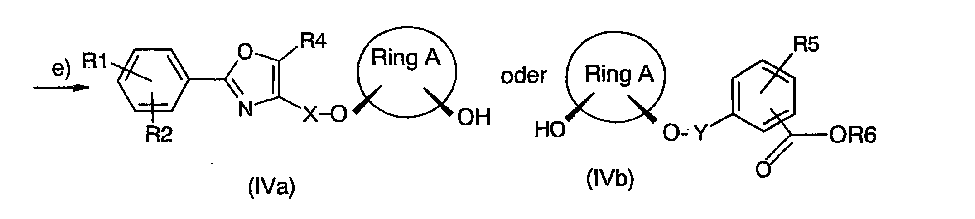

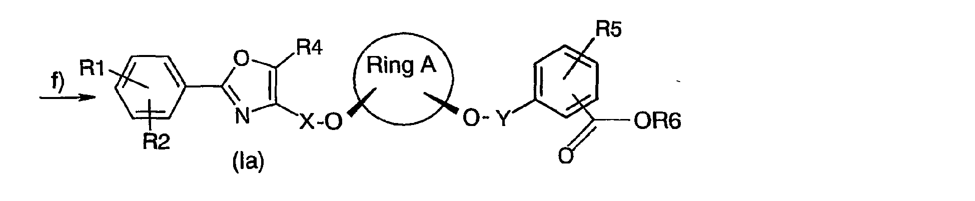

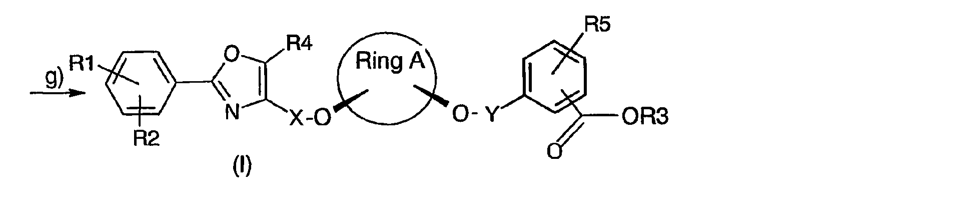

Die in Schema I aufgeführten Verfahrensschritte werden nachfolgend nochmals detailliert erläutert.

Bei den in Schema I aufgeführten Verbindungen (I bis VIII) liegt bezüglich der beiden am Ring A (in den jeweiligen Verbindungen) angebundenen Substituenten eine cis-Substitution dieser beiden Substituenten in Bezug auf den Ring A vor. Beispielsweise kann es sich um eine cis-1,2-, cis-1,3- oder cis-1,4-Substitution handeln. Bevorzugt sind hierbei die cis-1,2- und die cis-1,3-Substitution und mehr bevorzugt die cis-1,3-Substitution. Besonders bevorzugt ist die cis-1,3-Substitution am Cyclohexyl-Ring A. Der Einfachheit halber werden der Ring A oder auch die Substituenten X und Y nachfolgend als einfache Radikale (Alkyle oder Alkenyle) bezeichnet, auch wenn - je nach Betrachtungsweise - im Fall des Ring A eine Bezeichnung als Alkan oder Alken (Ring A als Grundfragment der Formel (I)) beziehungsweise als Alkylen oder Alkenylen denkbar ist.

Als geeigneter Enantiomerenüberschuß (hohe Enantioselektivität) soll eine Enantiomerenreinheit (ee) von größer 50% ee, bevorzugt größer 90% ee, mehr bevorzugt größer 95% ee, noch mehr bevorzugt größer 98% ee, viel mehr bevorzugt größer 99% ee und besonders bevorzugt größer 99,5% ee verstanden werden.

Vorzugsweise werden die Schritte a1) und/oder a2) in Gegenwart der Lipase B aus Candida antarctica durchgeführt.

Das erfindungsgemäße Verfahren hat gegenüber denjenigen aus dem Stand der Technik die Vorteile, dass durch den Einsatz von geeigneten Enzymen die chirale Information bereits zu Beginn des Verfahrens in die jeweiligen Vorstufen eingeführt wird, wodurch diese Vorstufen bereits enantioselektiv in einem geeigneten, gegebenenfalls sogar in extrem hohen Enantiomerenüberschuß (Enantiomerenreinheit > 99 % ee) vorliegen. Folglich können auch die gewünschten Enantiomere der Verbindungen (I) enantioselektiv in einem geeigneten, gegebenenfalls sogar in extrem hohen Enantiomerenüberschuß (Enantiomerenreinheit > 99 % ee) hergestellt werden. Demzufolge ist gegenüber den aus dem Stand der Technik bekannten Verfahren kein Ausbeuteverlust von bis zu 50 % zu beobachten und es ist auch keine Auftrennung der racemischen Gemische der cis-Enantiomere der entsprechenden Zwischenverbindungen mehr erforderlich, um ein gewünschtes Enantiomere der Verbindungen (I) in einem geeigneten Enantiomerenüberschuß herzustellen.

Überraschenderweise bleibt die bereits zu Synthesebeginn gebildete chirale Information (teilweise > 99 % Enantiomerenreinheit der Vorstufen) aufgrund der unter basischen Bedingungen stabilen Schutzgruppe Z3 trotz zweier Alkylierungsschritte bis zum herzustellenden chiralen PPAR-Aktivator der Formel (I) erhalten, dessen Enantiomerenreinheit somit ebenfalls > 99 % ee ist. Weiterhin ermöglichen die im erfindungsgemäßen Verfahren eingesetzten Enzyme, dass die verwendeten Ausgangsmaterialien nicht nur in Form des jeweiligen reinen cis-Isomers, sondern auch als cis/trans-Gemische eingesetzt werden können, ohne dass dabei die Enantiomerenreinheit der Zwischenprodukte beziehungsweise des Zielmoleküls beeinträchtigt wird. Sofern im erfindungsgemäßen Verfahren cis/trans-Gemische der Ausgangsmaterialien eingesetzt werden, lassen sich die entsprechenden trans-Ausgangsverbindungen aufgrund der verwendeten Schutzgruppentechnik beim Aufreinigen der Zwischenprodukte, beispielsweise durch Extraktion, problemlos entfernen. Zusätzliche Aufreinigungsschritte, beispielsweise mit Chromatographie, sind hierfür nicht extra erforderlich.

Gegenüber der in WO 03/020269 offengelegten Syntheseroute können insbesondere folgende Vorteile hervorgehoben werden: Bei Auswahl einer geeigneten Lipase kann nahezu reines Enantiomer (>99%ee) der Verbindung (V) durch die enzymatische Desymmetrisierung gebildet werden, welches als chirales Ausgangsmaterial ein wichtiger Baustein für die stereoselektive Synthese der PPAR-Aktivatoren der Formel (I) mit optischen Reinheiten > 99 % ee ist; die stereochemische Information bleibt überraschenderweise mit Hilfe einer geeigneten Schutzgruppentechnik bis zum herzustellenden chiralen PPAR-Aktivator erhalten, so dass nicht mehr die Hälfte der wertvollen Ausgangsmaterialien als Abfall zu entsorgen ist; eine aufwendige Racemattrennung z.B. mit Hilfe einer chiralen Chromatographie muss nicht mehr durchgeführt werden; Einsatz und Entsorgung von giftigen Zinnverbindungen, lodid-haltigen Verbindungen und Cäsiumfluorid sind nicht mehr notwendig; Einsatz von Natriumhydrid als Base und N,N-Dimethylformamid als Lösungsmittel ist nicht mehr notwendig; Chromatographien sind, wenn überhaupt, nur noch in einem geringen Ausmaß erforderlich.

Insbesondere mit der in Schritt a1) des erfindungsgemäßen Verfahrens verwendeten Lipase B aus Candida antarctica können bei einem Umsatz von > 90 % bereits in Lösung Enantiomerenreinheiten von > 99 % ee erzielt werden, ohne dass hierfür eine Umkristallisation erforderlich ist.

Mit dem erfindungsgemäßen Verfahren lassen sich Verbindungen der allgemeinen Formel (I) herstellen, 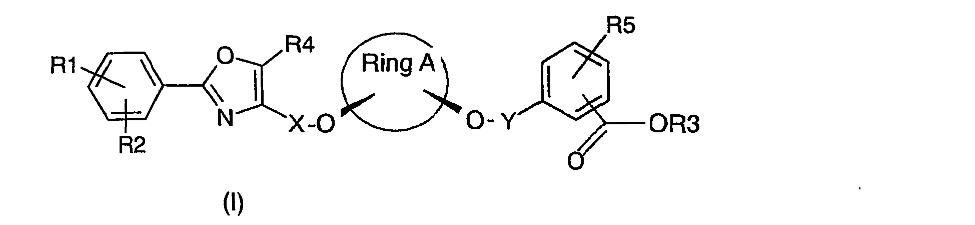 worin:
Ring A ist C₃-C₈-Cycloalkyl oder C₃-C₈-Cycloalkenyl, wobei in den Cycolalkyl-oder Cycloalkenylringen ein oder mehrere Kohlenstoffatome durch Sauerstoffatome ersetzt sein können,
R1, R2, R4 und R5 sind unabhängig voneinander H, F, Cl, Br, OH, NO₂, CF₃, OCF₃, C₁-C₆-Alkyl oder -O-(C₁-C₆-Alkyl),
R3 ist H, C₁-C₆-Alkyl oder Benzyl, die gegebenenfalls mit F, Cl, Br, OH, NO₂, CF₃, OCF₃, C₁-C₆-Alkyl oder -O-(C₁-C₆-Alkyl) substituiert sein können,
X ist C₁-C₆-Alkyl, wobei in der Alkylgruppe ein oder mehrere Kohlenstoffatome durch Sauerstoffatome ersetzt sein können,
Y ist C₁-C₆-Alkyl, wobei in der Alkylgruppe ein oder mehrere Kohlenstoffatome durch Sauerstoffatome ersetzt sein können.

Bevorzugt lassen sich mit dem erfindungsgemäßen Verfahren Verbindungen der allgemeinen Formel (I) herstellen, worin:
Ring A ist Cyclopentyl, Cyclohexyl oder Cycloheptyl,
R1, R2, R4 und R5 sind unabhängig voneinander H, F, Cl, Br, OH, NO₂, CF₃, - OCF₃, C₁-C₆-Alkyl oder O-C₁-C₆-Alkyl,
R3 ist H oder C₁-C₆-Alkyl oder Benzyl
X und Y sind unabhängig voneinander C₁-C₆-Alkyl,

Mehr bevorzugt lassen sich mit dem erfindungsgemäßen Verfahren Verbindungen der allgemeinen Formel (I) herstellen, worin:
Ring A ist Cyclohexyl, wobei der X-enthaltende und der Y-enthaltende Substituent von Formel (I) in cis-1,3-Position zum Cyclohexyl-Fragment stehen,
X und Y sind Methyl.
Noch mehr bevorzugt lassen sich mit dem erfindungsgemäßen Verfahren Verbindungen der allgemeinen Formel (I) herstellen, worin
Ring A ist Cyclohexyl, wobei der X-enthaltende und der Y-enthaltende Substituent von Formel (I) in cis-1,3-Position zum Cyclohexyl-Fragment stehen und das Kohlenstoffatom des Ringes A, das mit dem Y-enthaltenden Substituenten substituiert ist, R-Konfiguration aufweist.
X und Y sind Methyl.
Besonders bevorzugt lassen sich mit dem erfindungsgemäßen Verfahren Verbindungen der allgemeinen Formel (I) herstellen, worin
Ring A ist Cyclohexyl, wobei der X-enthaltende und der Y-enthaltende Substituent von Formel (I) in cis-1,3-Position zum Cyclohexyl-Fragment stehen und das Kohlenstoffatom des Ringes A, das mit dem Y-enthaltenden Substituenten substituiert ist, R-Konfiguration aufweist.
X und Y sind Methyl.
R1, R2 und R4 sind unabhängig voneinander H, F, Cl, C₁-C₃ Alkyl oder -O-(C₁-C₃-Alkyl),
R5 ist H oder C₁-C₃-Alkyl.

Die in Schema I aufgeführte Verbindung (IX), , kann wiederum hergestellt werden durch:
i) Umsetzung der Verbindung (X) mit mindestens einem Acyldonor in Gegenwart eines Enzyms, das hauptsächlich das cis-Isomere der Verbindung (IX) liefert und die gegebenenfalls als Nebenprodukte entstehenden trans-Isomere der Verbindungen der Formel (V) abgetrennt werden, oder
ii) Umsetzung der Verbindung (X) mit mindestens einem Acyldonor.

Vorzugsweise wird Schritt i) in Gegenwart einer Lipase der Schweinepankreas, einer Lipase aus Burkholderia cepacia, einer Lipase aus Burkholderia species, einer Lipase aus Pseudomonas cepacia oder einer Lipase aus Pseudomonas species durchgeführt.

Das Verfahren wird unter Bezugnahme auf Schema I im Rahmen einer als typisch - einschließlich der Vorstufen - einzusetzenden Reaktionsfolge nachstehend exemplarisch dargestellt.

Sofern im nachstehenden Text eine Bezugnahme auf ein konkretes Beispiel erfolgt, dient dies alleine zur Verdeutlichung der hier exemplarisch beschriebenen Reaktionsfolge und bedeutet nicht, dass das erfindungsgemäße Verfahren auf das konkrete Beispiel beschränkt ist.

### Schritt a)

Im Verfahren werden durch Schritt a) die Verbindungen (V) hergestellt, wobei hierfür mehrere Wege offen stehen. Die Verbindungen (V) sind bereits seit längerem in der Literatur bekannt. Beispielsweise werden von K. Dimroth et al., Ber. (1942), 75B, 322-6 das Monoacetat von Acyloxycyclohexanolen beschrieben. In der eingangs zitierten Veröffentlichung von T. Hirata et al. wird zudem beschrieben, wie diverse cis-1S-Acyloxycyclohexan-3R-ole durch chirale Chromatographie isoliert werden können. Das Hauptproblem ist hierbei in der Auftrennung der Verbindung (V) in die einzelnen Enantiomere zu sehen, was in der Praxis sehr große Schwierigkeiten bereitet, da hierfür hauptsächlich chirale Chromatographie eingesetzt werden muss.

### a) Enzymatische Acylierung

Als Alternative zur chiralen Chromatographie können die einzelnen Enantiomere der Verbindungen (V) durch enzymatische Acylierung aus Verbindungen (X) dargestellt werden. Die Verbindungen (X) können entweder als cis/trans-Isomerengemische oder als reine cis-Isomere eingesetzt werden und sind in diesen Formen von diversen Anbietern (beispielsweise von den Firmen Merck, Fluka oder Aldrich) kommerziell erhältlich. Sofern die Verbindungen (X) in Form des reinen cis-Isomeren eingesetzt werden, hat dies den Nachteil, dass diese erst aus den entsprechenden cis/trans-Gemischen isoliert werden müssen beziehungsweise dass die reinen cis-Isomere teurer sind. Zur Auftrennung von cis/trans-Gemischen von Verbindungen (X) kann beispielsweise im Fall von 1,3-Cyclohexandiol eine Kristallisation als cis-1,3-Cyclohexandiol-Kupferkomplex (W. Rigby, J. Chem. Soc. (1949), 1586; R. Sillanpää et al., Polyhetron 21 (2002), 1133-1138) benutzt werden.

Ausgehend von Verbindungen (X) kann die enzymatische Acylierung im Rahmen des erfindungsgemäßen Verfahrens in Gegenwart von verschiedenen Enzymen ( beispielsweise Lipasen) mit einem Acyldonor durchgeführt werden. Hierbei kann ein einzelner Acyldonor oder ein Gemisch von mehreren Acyldonoren eingesetzt werden. Die Reaktion kann entweder ohne Zusätzliches organisches Lösungsmittel (Beispiel 1) oder mit einem zusätzlichen organischen Lösungsmittel (Beispiel 2) erfolgen. Als organische Lösungsmittel kommen hierfür prinzipiell alle gängigen organischen Lösungsmittel in Betracht wie Toluol, chlorierte Kohlenwasserstoffe oder Ether, vorzugsweise Methyl-tert.-butylether. Die Reaktion kann jedoch nicht in Wasser durchgeführt werden. Als Acyldonor eignen sich alle chemischen Verbindungen, die eine säurestabile Schutzgruppe Z1 beziehungsweise Z2 ausbilden können. Beispielhaft aufgeführt sind hierfür Carbonsäureester. Bevorzugt geeignet hierfür sind Vinylester wie Vinylacetat, Isopropenylacetat, Vinyllaurat oder Vinylbutyrat, besonders bevorzugt Vinylacetat oder Isopropenylacetat.

Für die enzymatische Acylierung kann nicht jedes beliebige Enzym eingesetzt werden. Es muss vielmehr ein Enzym verwendet werden, mit dem das gewünschte Zielmolekül entweder direkt oder indirekt in einem geeigneten Enantiomerenüberschuß hergestellt werden kann. Weiterhin hängt es auch von der verwendeten Enzym ab, ob aus den Verbindungen (X) die Verbindungen (V) direkt gebildet werden oder ob (indirekt) zuerst die Verbindung (IX) gebildet wird, die wiederum anschließend zu Verbindung (V) umgesetzt werden muss. Sofern eine Lipase eingesetzt wird, die aus der Fraktion B des Organismus Candida antarctica (nachfolgend als Lipase B aus Candida antarctica bezeichnet, Auftrennung der Fraktionen gemäß EP-A 287 634) stammt, wird ausgehend von einem cis/trans-Isomerengemisch der Verbindung (X) bevorzugt die cis-Monoacylverbindung (V) gebildet, während die Diacylverbindung (IX) nur als Nebenprodukt anfällt. Bei der Verwendung der Lipase B aus Candida antarctica fällt hingegen keine trans-Monoacylverbindung (V) an, weil die entsprechende trans-Ausgangsverbindung (X) entweder nicht oder zur entsprechenden Diacylverbindung (IX) umgesetzt wird.

Die direkte Umsetzung (enzymatische Acylierung) einer Verbindung der Formel (X) zu einer Verbindung der Formel (V) wird vorzugsweise in Gegenwart der Lipase B aus Candida antarctica durchgeführt. Besonders bevorzugt wird diese Umsetzung in Gegenwart einer Lipase ausgewählt aus Chirazyme L2 lyo., Chirazyme L2 c.f. C2 oder Chirazyme L2 c.f. C3 durchgeführt. Die Zuordnung der vorgenanten Enzyme (in Form ihres Handelsnamens) zu der zugehörigen Accession-Nummer der Genbank des National Center for Biotechnology Information (NCBI) kann der in Beispiel 1 aufgeführten Tabelle 1 entnommen werden.

Das bei dieser Umsetzung anfallende Reaktionsgemisch an Verbindungen der Formeln (IX) und (V) kann gegebenenfalls durch Extraktion, Destillation oder Chromatographie getrennt werden. Um einen Enantiomerenüberschuß zu erzielen, ist die Auftrennung jedoch an dieser Stelle nicht zwingend erforderlich, weil die als Nebenprodukt gebildete Verbindung (IX) im darauf folgenden Schritt b) nicht mit einer Schutzgruppe Z3 versehen werden kann. Demzufolge wird das Nebenprodukt (IX) in Schritt c) des erfindungsgemäßen Verfahrens doppelt entschützt und gegebenenfalls bei der Aufarbeitung der Verbindung (II) durch Extraktion entfernt. Eine ähnliche Betrachtung kann für das nicht umgesetzte Edukt (X) angestellt werden, dass entweder bereits in diesem Verfahrensschritt durch Extraktion, Destillation oder Chromatographie abgetrennt werden kann beziehungsweise bei der Aufarbeitung der Verbindungen (II) oder (IX).

Anstelle der Lipase B aus Candida antarctica können bei der enzymatischen Acylierung auch eine Lipase der Schweinepankreas, eine Lipase aus Burkholderia cepacia, eine Lipase aus Burkholderia species, eine Lipase aus Pseudomonas cepacia oder eine Lipase aus Pseudomonas species verwendet werden. Sofern diese Lipasen eingesetzt werden, werden aus dem Edukt (X) sowohl Verbindungen (IX) als auch (V) gebildet. Die Monoacylverbindungen (V) liegen jedoch in der nicht gewünschten trans-Form vor, während die ebenfalls gebildeten Verbindungen (IX) überraschenderweise überwiegend als cis-Diacyl vorliegen. Diese cis-Diacylverbindungen (IX) können wie nachfolgend erläutert durch enzymatische Desymmetrisierung (enzymatische Hydrolyse) zu den gewünschten cis-Enantiomeren der Verbindung (V) umgesetzt werden.

Die Umsetzung einer Verbindung (X) durch enzymatische Acylierung zu einer Verbindung (IX), die hauptsächlich als cis-Isomer vorliegt, wird vorzugsweise in Gegenwart einer Lipase ausgewählt aus einer Lipase der Schweinepankreas, eine Lipase aus Burkholderia cepacia, einer Lipase aus Burkholderia species, einer Lipase aus Pseudomonas cepacia oder einer Lipase aus Pseudomonas species durchgeführt. Mehr bevorzugt ist die Lipase ausgewählt aus einer Lipase der Schweinepankreas, eine Lipase aus Burkholderia cepacia, einer Lipase aus Burkholderia species oder Pseudomonas cepacia. Viel mehr bevorzugt ist die Lipase ausgewählt aus Chirazyme L1 lyo, Chirazyme L1 c.f., Chirazyme L7 lyo oder Lipase PS. Besonders bevorzugt ist die Lipase ausgewählt aus Chirazyme L1 lyo., Chirazyme I1 c.f. oder Chirazyme I7 lyo. Die Zuordnung der vorgenannten Enzym-Handelsnamen zu der NCBI-Accession-Nummer kann Tabelle 1 entnommen werden.

Um einen Enantiomerenüberschuß zu erzielen, ist es allerdings erforderlich, dass die bei diesem Syntheseweg als unerwünschtes Nebenprodukt gebildete trans-Verbindung (V) von der Verbindung (IX) durch Extraktion, Destillation oder gegebenenfalls Chromatographie abgetrennt wird, sofern das Edukt (X) als cis/trans-Gemisch eingesetzt wird. Dieser Aufarbeitungsschritt entfällt jedoch, sofern die Verbindung (X) als reines cis-Isomer eingesetzt wird. Bevorzugt wird die etwaige Abtrennung des Nebenproduktes (V) per Extraktion durchgeführt. Da die anschließende enzymatische Desymmetrisierung der Verbindung (IX) mit einem anderen Enzym sowie in wässriger Phase durchgeführt wird, sollte vorher das bei der enzymatischen Acylierung verwendete Enzym beispielsweise durch Filtration entfernt werden. Das Enzym wird vorzugsweise vor der Abtrennung der Monoacylverbindung (V-trans) entfernt.

### Chemische Acylierung/enzymatische Desymmetrisierung

Ein weiterer möglicher Ausgangspunkt zur Darstellung der Verbindungen (V) sind die Verbindungen (IX), die ebenfalls in Form von cis/trans-Isomerengemischen beziehungsweise als reines cis-Isomer von diversen Anbietern (beispielsweise die Firmen Merck, Fluka oder Aldrich) kommerziell erhältlich sind. Die Auftrennung von cis/trans-Isomerengemischen kann beispielsweise im Fall von cis-1,3-Diacetoxycyclohexan durch den um 1 °C unterschiedlichen Siedepunkt der beiden Isomere per Destillation erfolgen. Aufgrund der oft geringen Siedepunktdifferenzen ist dieses Verfahren jedoch aufwendig und kostspielig. Die Verbindungen (IX) können wie vorstehend erwähnt durch enzymatische Acylierung aus den Verbindungen (X) gewonnen werden. Alternativ können die Verbindungen (X) auch direkt mit den vorstehenden Acyldonoren (in Abwesenheit von Enzymen) zu den Verbindungen (IX) umgesetzt werden. Diese Reaktion ist bereits seit langem bekannt und wird als chemische Acylierung bezeichnet, die jedoch nicht stereoselektiv abläuft (Beispiele 3 und 4). Die chemische Acylierung kann beispielsweise mit Essigsäureanhydrid/4-Dimethylaminopyridin (4-DMAP), Triethylamin (TEA) in Dichlormethan durchgeführt werden. Die chemische Acylierung kann entweder mit einem einzelnen Acyldonor oder einem Acyldonorengemisch durchgeführt werden, bevorzugt ist die Verwendung eines einzelnen Acyldonors, so dass in der Verbindung (IX) die Substituenten Z1 und Z2 die gleiche Bedeutung haben.

Die Verbindungen (IX) können mit Wasser zu der Verbindung (V) in Gegenwart eines Enzyms, das einen geeigneten Enantiomerenüberschuß der Verbindung (V) liefert, umgesetzt werden. Vorzugsweise wird als Enzym die Lipase B aus Candida antarctica verwendet. Besonders bevorzugt wird diese Umsetzung in Gegenwart einer Lipase ausgewählt aus Chirazyme L2 lyo., Chirazyme L2 c.f. C2 oder Chirazyme L2 c.f. C3 durchgeführt. Diese Reaktion muss in wässriger Lösung durchgeführt werden, die ausschließliche Verwendung von organischen Lösungsmitteln ist hier nicht geeignet. Überraschenderweise wird die trans-Diacylverbindung (IX) von der Lipase B aus Candida antarctica nicht umgesetzt.

Folglich kann mit diesen beiden Methoden (enzymatische Acylierung und chemische Desymmetrisierung) sowie der ebenfalls möglichen Kombination dieser beiden Methoden (enzymatische Acylierung mit anschließender enzymatischer Desymmetrisierung) ein cis/trans-Isomerengemisch der Verbindungen (X) zur Herstellung einem Enantiomerenüberschuß beziehungsweise einer enantiomerenreinen cis-Monoacylverbindung (V) eingesetzt werden. Dieses Verfahren ist kostengünstiger als die Verwendung des reinen cis-Isomeren (X). Die Herstellung von enantiomerenreinen Verbindungen (V) ist somit ein weiterer Gegenstand der vorliegenden Erfindung. Als enantiomerenreine Verbindungen sollen im Rahmen der vorliegenden Erfindung Verbindungen verstanden werden, die einen Reinheitsgrad von > 98 % (ee > 98 %), bevorzugt > 99 % (ee > 99 %), besonders bevorzugt > 99,5 % (ee > 99,5 %) aufweisen.

Der große Vorteil bei der Verwendung der Lipase B aus Candida antarctica ist darin zu sehen, dass unabhängig davon, ob ein einzelner Acyldonor oder ein Gemisch von Acyldonoren verwendet wird, immer die Verbindung (V) in enantiomerenreiner Form ausgebildet wird. In den Verbindungen (IX), (V) und (XIII) sind die Schutzgruppen Z1 und Z2 unabhängig voneinander eine säurestabile Schutzgruppe. Vorzugsweise weisen die Schutzgruppen Z1 und Z2 die gleiche Bedeutung auf. Z1 und Z2 sind bevorzugt -C(O)-R, R ist gegebenenfalls substituiertes Alkyl oder Aryl, beispielsweise C₁-C₆-Alkyl oder Phenyl. Z1 und Z2 sind mehr bevorzugt unabhängig voneinander -C(O)-(C₁-C₃-Alkyl), besonders bevorzugt -C(O)-CH₃. Die Lipase B aus Candida antarctica kann sowohl in ihrer nicht immobilisierten Form (Chirazym L2) als auch in ihren immobilisierten Formen (c.f., c.f.C2, c.f.C3, Hersteller: Roche Diagnostics) eingesetzt werden.

Die Lipase B aus Candida antarctica ist auch von anderen Herstellern wie z.B. Novozymes (Novozym 435 als Immobilisat) erhältlich. Alternativ hierzu kann auch gelöste Lipase B aus Candida antarctica wie z.B. Novozym CALB L oder Novozym 525 F nach Immobilisierung des Enzyms verwendet werden.

Die vorstehend beschriebenen Trennverfahren von cis/trans-Gemischen der Verbindungen (X) oder (IX) beziehungsweise das Herstellungsverfahren eines Enantiomerenüberschußes einer cis-Verbindung (V) oder einer enantiomerenreinen cis-Verbindung (V) finden erfindungsgemäß insbesondere Anwendung, indem der Enantiomerenüberschuß einer cis-Verbindung (V) oder eine enantiomerenreine cis-Verbindung (V) durch eine geeignete Schutzgruppentechnik und weitere Alkylierungsschritte in die gewünschten Zielmolekül (I) ( im Enantiomerenüberschuß oder enantiomerenrein) überführt werden, wobei weder eine chirale noch eine achirale Chromatographie notwendig ist.

Alle Versuche, eine selektive O-Alkylierung der enantiomerenreinen *Verbindung (V)* zu erreichen, sind bislang gescheitert, da eine inter- und intramolekulare Wanderung der Acylgruppe unter den nicht zu vermeidbaren basischen Alkylierungsbedingungen zu beobachten war (Acyl = z.B. Acetyl, Benzoyl). Es wird deshalb versucht, eine basenstabile Schutzgruppentechnik wie z.B. Tetrahydropyranyl, Methoxyisopropyl als Schutzgruppe zu verwenden, so dass die chirale Information, die durch die enzymatische Desymmetrisierung in Verbindung (V) generiert worden ist, trotz der basischen Alkylierungsbedingungen erhalten bleibt. Durch eine gezielte Alkylierungsreihenfolge bzw. Schutzgruppenstrategie kann als weiterer Gegenstand der vorliegenden Erfindung die Herstellung des gewünschten stereoisomeren PPAR-Aktivators überraschenderweise ohne Verlust der chiralen Information erreicht werden.

### Schritt b)

Die Verbindung (V) wird in Gegenwart eines sauren Katalysators mit einer Verbindung, die eine basenstabile und säurelabile Schutzgruppe Z3 ausbilden kann, zur Verbindung der Formel (VIII) umgesetzt. Als saure Katalysatoren können beispielsweise anorganische Säuren, Toluolsulfonsäure, Pyridinium-paratöluolsutfonat oder saure Ionenaustauscher wie Amberlyst H15 verwendet werden. Vorzugsweise wird hierfür Pyridinium-para-toluolsulfonat eingesetzt. Die in der Verbindung (VIII) vorhandene Schutzgruppe Z3 ist eine basenstabile und säurelabile Acetal-oder Ketalschutzgruppe. Z3 ist mehr bevorzugt Tetrahydropyranyl oder Methoxyisopropyl, besonders bevorzugt Tetrahydropyranyl. Als Verbindung, die die basenstabile und säurelabile Schutzgruppe Z3 ausbilden kann, eignet sich vorzugsweise 3,4-Dihydro-2H-pyran. Ein Äquivalent der Verbindung (V) wird mit 1 bis 10 Äquivalenten der die basenstabile und säurelabile Schutzgruppe Z3 ausbildenden Verbindung umgesetzt, bevorzugt mit 1,1- bis 1,4 Äquivalenten. Der saure Katalysator wird in der Regel mit 0,01 bis 1 Äquivalent, bevorzugt mit 0,05 bis 0,1 Äquivalent eingesetzt. Die Reaktionstemperatur beträgt gewöhnlicherweise 20 bis 80°C, bevorzugt 50 bis 60°C. Schritt b) wird wie alle anderen Schritte dieses Verfahrens gewöhnlich bei Normaldruck durchgeführt. Als Lösungsmittel für Schritt b) eignen sich organische Lösungsmittel wie zum Beispiel chlorierte Kohlenwasserstoffe, Carbonsäureester wie Essigester, Carbonsäureamide wie N-Methylpyrrolidon, Etherverbindungen wie Diethylether oder Methyl-tert.-butylether, aromatische Kohlenwasserstoffe wie Chlorbenzol oder Toluol. Alternativ kann auch 3,4-Dihydro-2H-pyran selbst als Lösungsmittel verwendet werden. Bevorzugtes Lösungsmittel ist Toluol. Wasser oder Alkohole kommen hingegen als Lösungsmittel nicht in Frage, da diese beispielsweise mit 3,4-Dihydro-2H-pyran zu den entsprechenden Acetalen abreagieren. Die in diesem Schritt ausgebildete Verbindung (VIII) kann zur Reinigung destilliert werden, sie kann jedoch ohne weitere Aufreinigung im nächsten Verfahrensschritt eingesetzt werden.

### Schritt c)

Die Verbindung (VIII) wird in Gegenwart eines Nukleophils zu der Verbindung (II) umgesetzt. Für diese als Deacylierung bezeichnete Reaktion kann als Nukleophil beispielsweise ein Alkali- oder Erdalkalialkoholat, vorzugsweise Natriummethanolat eingesetzt werden. Auf ein Äquivalent der Verbindung (VIII) werden 0,1 bis 10 Äquivalente Nukleophil verwendet, bevorzugt sind katalytische Mengen von 0,1 bis 0,3 Äquivalenten. Die Reaktionstemperatur beträgt gewöhnlich 10 bis 80°C, bevorzugt 15 bis 25°C. Dieser Deacylierungsschritt kann in allen organischen Lösungsmitteln ausgeführt werden, die nicht mit dem Nukleophil (Natriummethanolat) reagieren, zum Beispiel aromatische Kohlenwasserstoffe, Alkohole, chlorierte Kohlenwasserstoffe. Hierbei ist Toluol als Lösungsmittel bevorzugt, da auch im vorausgehenden Verfahrensschritt mit Toluol extrahiert werden kann, so dass kein Lösungsmittelwechsel bei der Deacylierung notwendig ist, mit Toluol kann im Nachfolgenden Schritt d) ebenfalls die Alkylierung durchgeführt werden. Die Verbindung (II) kann zur Reinigung destilliert werden, was aber nicht zwingend erforderlich ist.

### Schritt d)

Die Verbindung (II) wird in Gegenwart einer Base B1 mit einer Verbindung der Formel (VI) zu einer Verbindung der Formel (IIIa) oder mit einer Verbindung der Formel (VII) zu einer Verbindung der Formel (IIIb) umgesetzt. Als Basen B1 eignen sich tertiäre Erdalkalialkoholate, tertiäre Alkalialkoholate, Erdalkaliamide, Alkaliamide, Erdalkalisilazide, Alkalisilazide oder Alkalihydride. Primäre oder sekundäre Alkoholate sind hingegen nicht geeignet. Bevorzugte Basen B1 sind Kalium-tert.-butylat (KOtBu), tertiäres Isopentylat, Lithiumdiisopropylamid oder Kalium-bis-(trimethylsilyl)-amid. Besonders bevorzugt sind Kalium-tert.-butylat oder Kalium-bis-(trimethylsilyl)-amid. Als Lösungsmittel eignen sich organische aprotische Lösungsmittel, beispielsweise Etherverbindungen (Diethylether, Methyl-tert.-butylether), Carbonsäureamide (N-Methylpyrrolidon), aromatische Kohlenwasserstoffe (Chlorbenzol oder Toluol), bevorzugt ist hierbei Toluol. Die Reaktion wird normalerweise bei 20 bis 80°C, bevorzugt bei 50 bis 60°C durchgeführt. Hierbei wird normalerweise 1 Äquivalent der Verbindung (II) mit 1 bis 3 Äquivalenten an Alkylierungsmittel (Verbindungen (VI) beziehungsweise (VII)), bevorzugt 1,1 bis 1,3 äquivalente Alkylierungsmittel umgesetzt. Die Base B1 wird mit 1 bis 3, bevorzugt 1,5 bis 2 Äquivalenten verwendet.

Die Alkylierungsreagenzien der Formeln (VI) beziehungsweise (VII) sind kommerziell erhältlich oder können nach literaturbekannten Methoden hergestellt werden. Z4 und Z5 sind unabhängig voneinander eine Abgangsgruppe. Hierbei können alle gängigen Abgangsgruppen verwendet werden, bevorzugt sind Chlor oder Brom. Herstellungsverfahren zu Verbindungen der Formel (VI) finden sich beispielsweise in der WO 03/020269 oder in der internationalen Anmeldung WO 2004/076390 beziehungsweise in The Chemistry of Heterocyclic Compounds (Ed.: A. Weissberger, E. C. Taylor): Oxazoles (Ed.: I.J. Turchi), b). Methoden der Organischen Chemie, Houben-Weyl 4. Auflage., Hetarene III, Teilband 1; c) I. Simit, E. Chindris, Arch. Pharm. 1971, 303, 425; d). Y. Goto, M. Yamazaki, M. Hamana, Chem. Pharm. Bull. 1971, 19 (10), 2050-2057]. Die Verbindungen der Formel (VII) sind ebenfalls in den beiden vorgenannten Anmeldungen beschrieben sowie in WO 00/64888 (Isobutylester) und WO 00/64876 (Methylester). Weiterhin können diese Verbindungen durch radikalische Seitenkettenhalogenierung (s. Literatur-Übersicht R.C. Larock, Comprehensive Organic Transformations, S. 313, 1989 VCH Publishers, Inc.) oder aus den Alkoholen beziehungsweise daraus herstellbaren Derivaten (s. Literatur-Übersicht R.C. Larock, Comprehensive Organic Transformations, S. 353-363., 1989 VCH Publishers, Inc.) hergestellt werden. Bekannt ist weiterhin (s. J. Chem. Soc. 1925, 127, 2275-2297; J: Chem. Soc. 1922, 121, 2202-2215) die Herstellung verschiedener 2-Bromomethylbenzoesäurebromide durch radikalische Bromierung, die dann durch weitere Umsetzung mit Alkoholen in die zur Gruppe der Alkylierungsreagenzien der Formel III gehörenden Bromomethylbenzoesäureester überführt werden können.

Die Entscheidung, ob in Schritt d) mit der Verbindung (VI) oder der Verbindung (VII) alkyiert wird, richtet sich nach dem als Zielmolekül (I) gewünschten Enantiomer. Vorzugsweise werden die Verbindungen (II) mit der Verbindung der Formel (VI) umgesetzt, insbesondere wenn der Ring A cis-1,3-Cyclohexyl ist.

### Schritt e)

Die Verbindung (IIIa) wird zu der Verbindung (IVa) oder die Verbindung (IIIb) zu der Verbindung (IVb) umgesetzt, wobei die jeweilige Umsetzung mit einem Alkohol in Gegenwart eines sauren Katalysators erfolgt. Als saure Katalysatoren eignen sich die gleichen Verbindungen, die bereits in Schritt b) aufgeführt sind, wobei die Auswahl des sauren Katalysators in Schritt b) und e) unabhängig voneinander erfolgen kann. Als Alkohole eignen sich vorzugsweise primäre Alkohole, insbesondere Methanol. Dieser Schritt wird bei einer Temperatur von 20 bis 80°C, bevorzugt 45 bis 55°C durchgeführt. Als Lösungsmittel eignen sich die bereits unter Schritt d) aufgeführten organischen aprotischen Lösungsmittel, vorzugsweise Toluol. Die Auswahl des Lösungsmittels in Schritt d) und e) kann unabhängig voneinander erfolgen. Ein Äquivalent der Verbindungen (III) wird mit 0,01 bis 10 Äquivalenten Säure, vorzugsweise 0,05 Äquivalente von beispielsweise Salzsäure umgesetzt. Die verwendeten Alkohole werden hierbei mit 1 bis 3 Äquivalenten eingesetzt. Die in diesem Schritt ausgebildete Verbindung (IV) kann zur Reinigung destilliert werden. Sofern diese Verbindung kristallin ist, wird eine Reinigung durch Kristallisation bevorzugt, weniger bevorzugt ist eine Reinigung mittels Chromatographie, da hierfür ein zu hoher Einsatz an Lösungsmittel notwendig ist.

### Schritt f)

Die Verbindung (IVa) wird in Schritt f) mit der Verbindung (VII) oder die Verbindung (IVb) mit der Verbindung (VI) zu der Verbindung (la) in Gegenwart der Base B1 umgesetzt. Die Auswahl der Base B1 erfolgt dabei unabhängig zu Schritt d), vorzugsweise wird jedoch die gleiche Base wie in Schritt d) eingesetzt. Prinzipiell können auch die gleichen Lösungsmittel wie in Schritt d) verwendet werden, wobei die Lösungsmittelwahl ebenfalls unabhängig von Schritt d) erfolgt. Vorzugsweise kann als Lösungsmittel neben Toluol auch Chlorbenzol bei diesem zweiten Alkylierungsschritt ausgewählt werden, wobei Chlorbenzol hier aufgrund eines größeren Umsatzes gegenüber Toluol bevorzugt ist. Die eingesetzten Mengenverhältnisse an Ausgangsverbindungen, Alkylierungsmittel sowie Base B1 entsprechen denjenigen von Schritt d). Die Reaktion wird gewöhnlich bei -30 bis +20°C, bevorzugt bei -5 bis +5°C durchgeführt.

### Schritt g)

Dieser Schritt ist nur erforderlich, sofern im Zielmolekül der Formel (I) der Rest R3 Wasserstoff ist, d.h. der gewünschte PPAR-Aktivator soll in Form der freien Säure vorliegen. Andernfalls entspricht die in Schritt f) erhaltene Verbindung (Ia) der Verbindung (I). Wenn dies jedoch nicht der Fall ist, wird die Verbindung (Ia) durch Hydrolyse oder Hydrogenolyse in die Verbindung (I) überführt. Die Hydrolyse kann nach gängigen Verfahren sowohl im basischen (R6 ist vorzugsweise n-Alkyl) oder im sauren (R6 ist vorzugsweisen tert.-Butyl) durchgeführt werden. Sofern R6 ein Benzylrest ist, wird die Verbindung (I) vorzugsweise durch eine Hydrogenolyse mit dem Fachmann bekannten Methoden erhalten. Im Fall der basischen Verseifung werden Metallhydroxide wie zum Beispiel Alkali- oder Erdalkalihydroxide im Verhältnis von 1 bis 10 Äquivalenten zur zu verseifenden Verbindung eingesetzt. Als Lösungsmittel eignen sich Wasser, Alkohole oder weitere organische Lösungsmittel wie beispielsweise Etherverbindungen (Diethylether, Methyl-tert.-butylether), Carbonsäureamide (N-Methylpyrrolidon) oder aromatische Kohlenwasserstoffe. Vorzugsweise wird tert.-Butanol verwendet. Die Reaktionstemperatur beträgt 20 bis 100°C, vorzugsweise 65 bis 75°C. Anschließend wird durch Ansäuern der Carboxylfunktion beispielsweise mit organischen oder anorganischen Säuren, vorzugsweise mit Salzsäure, der gewünschte chirale PPAR-Aktivator der Formel (I) in einer Enantiomerenreinheit von > 99 % ee freigesetzt. Gegebenenfalls kann auch eine Umkristallisation mit organischen Lösungsmitteln wie zum Beispiel aromatische Lösungsmittel, vorzugsweise Toluol, Essigester oder n-Butylacetat, oder gegebenenfalls mit Carbonsäureestern, Alkylethern oder Alkylalkoholen durchgeführt werden. Diese Umkristallisation kann ebenfalls im Anschluss an den Schritt e) durchgeführt werden.

Sofern die Schritte b) bis g) alle im gleichen Lösungsmittel durchgeführt werden, wird hierfür bevorzugt Toluol verwendet.

In einer bevorzugten Ausführungsform umfasst das erfindungsgemäße Verfahren die folgenden Schritte:
- a): eine Verbindung der Formel (IX) in Gegenwart der Lipase B aus Candida antarctica mit Wasser zu einer Verbindung der Formel (V) umgesetzt wird,
- b): die Verbindung (V) in Gegenwart eines saurer Katalysators mit einer Verbindung, die die basenstabile und saurelabile Acetal-oder Ketal- Schutzgruppe Z3 ausbilden kann, zur Verbindung der Formel (VIII) umgesetzt wird und
- c): die Verbindung (VIII) in Gegenwart eines Nukleophils zur Verbindung der Formel (II) umgesetzt wird,
- d): eine Verbindung (II) in Gegenwart einer Base B1 mit einer Verbindung der Formel (VI) zu einer Verbindung der Formel (IIIa) umgesetzt wird,
- e): die Verbindung (IIIa) zu einer Verbindung der Formel (IVa) umgesetzt wird, wobei die Umsetzung mit einem Alkohol in Gegenwart eines sauren Katalysators erfolgt,
- f): die Verbindung (IVa) mit der Verbindung (VII) zu einer Verbindung der Formel (la) in Gegenwart der Base B1 umgesetzt wird und
- g): gegebenenfalls die Verbindung (Ia) zu der Verbindung (I) hydrolysiert oder hydrogenolysiert wird, sofern R3 gleich H ist.

Diese bevorzugte Ausführungsform des erfindungsgemäßen Verfahrens eignet sich insbesondere zur Herstellung von Verbindungen der allgemeinen Formel (I), bei denen der Ring A gleich Cyclohexyl ist, wobei der X-enthaltende und der Y-enthaltende Substituent von Formel (I) in cis-1,3-Position zum Cyclohexyl-Fragment stehen und das Kohlenstoffatom des Ringes A, das mit dem Y-enthaltenden Substituenten substituiert ist, R-Konfiguration aufweist.

Alternativ kann in dieser bevorzugten Ausführungsform die Verbindung (V) durch enzymatische Acylierung mit einer geeigneten Lipase aus Verbindung (X) wie vorstehend beschrieben hergestellt werden, jedoch ist die Verbindung (IX) als Ausgangspunkt bevorzugt. Verbindung (IX) wiederum kann durch enzymatische Acylierung mit einer geeigneten Lipase aus Verbindung (X) oder bevorzugt über chemische Acylierung aus Verbindung (X) hergestellt werden.

Eine als typisch - einschließlich der Vorstufen - anzusetzende Reaktionsfolge ist für diese bevorzugten Ausführungsformen exemplarisch nachstehend dargestellt. Mit dieser Reaktionsfolge können insbesondere Verbindungen der Formel (I) hergestellt werden, bei denen der Ring A Cyclohexyl ist, die beiden X- und Y-enthaltenden Substituenten in cis-1,3-Position zum Ring A angeordnet sind und das Kohlenstoffatom des Ringes A, das mit den Y-enthaltenden Substituenten substituiert ist, R-Konfiguration aufweist. Die in den jeweiligen Synthesestufen beschriebenen Verfahrensbedingungen beispielsweise für die verwendeten Säuren, Basen oder Lösungsmittel gelten auch für die übrigen Verbindungen der Formel (I), bei denen der Ring A nicht auf cis-1,3-Cyclohexan-Derivate beschränkt ist.

Zusätzlich verdeutlicht das nachfolgende Schema (II) diese typische Reaktionsfolge zur Herstellung dieser Auswahl von PPAR-Aktivatoren. Ausgegangen wird dabei entweder von isomerenreinem cis-1,3-Cyclohexandiol (X-i-cis) (Fa. Clariant) oder von einem cis/trans Isomerengemisch (X-i) (Fa. Acros). Cis-1,3-Cyclohexandiol kann gegebenenfalls mit dem Fachmann bekannten Mitteln aus einem cis/trans-Cyclohexandiol-Isomerengemisch, beispielsweise durch Chromatographie, gewonnen werden. 1,3-Cyclohexandiol kann entweder durch eine enzymatische Acylierung direkt zu cis-1 S-Acyloxy-cyclohexan-3R-ol (V-i) oder durch chemische Acylierung über 1,3-Diacyloxy-cyclohexan (IX-i) als Zwischenstufe umgesetzt werden. Sowohl bei der chemischen als auch bei der enzymatischen Acylierung wird vorzugsweise nur einer der vorstehend beschriebenen Acyldonoren umgesetzt, so dass in Formel (IX-i) die säurestabile Schutzgruppen Z1 und Z2 dieselbe Bedeutung haben. 1,3-Diacyloxy-cyclohexan ist auch kommerziell als cis-Isomer (IX-1-cis) beziehungsweise als cis/trans Gemisch erhältlich (Fa. Clariant). Cis-1,3-Diacyloxy-cyclohexan beziehungsweise das cis/trans Gemisch (IX-i) wird dabei durch eine enzymatische Desymmetrisierung (enzymatische Hydrolyse) wie vorstehend beschrieben in das nahezu enantiomerenreine *cis*-1S-Acyloxy-cyclohexan-3R-ol (> 99 %ee) (V-i) umgewandelt. *cis*-1S-Acyloxy-cyclohexan-3R-ol wird daraufhin z.B. mit 3,4-Dihydro-2H-pyran oder Methoxypropen in Gegenwart von sauren Katalysatoren wie z.B. anorganischen Säuren, Toluolsulfonsäure, Pyridinium-para-toluolsulfonat oder saure Ionenaustauscher zu *cis*-1S-Acyloxy-3R-O-tetrahydropyranyl-cyclohexan (VIII-i mit Z3 = Tetrahydropyranyl) acetalisiert. Nach Deacylierung mit Nukleophilen wie z.B. organische Amine oder anorganische Erdalkali, Alkalihydoxide oder -alkoholate zu cis-O-Tetrahydropyranyl-cyclohexan-3S-ol (II-i) findet dann die Alkylierung mit einem Oxazolhalogenid (VI-i mit Z4 = Halogenid) in Anwesenheit einer der vorstehend beschriebenen anorganischen oder organischen Base zu *cis*-1S-O-Oxalyl-3R-O-tetrahydropyranyl-cyclohexan (IIIa-i) statt. An dieser Stelle wird darauf hingewiesen, dass im nachfolgenden Schema in den Formeln (IIIa-i; IVa-i, VI-i, VII-i, Ia-i sowie I-i) die beiden Variablen X und Y aus Gründen der Übersichtlichkeit als Methyl-Fragment aufgeführt sind. Dies ist jedoch keine Einschränkung, da diese Reaktionsfolge auch mit allen anderen Definitionen der Variablen X und Y ausführbar ist. *cis*-1 S-O-Oxalyl-3R-O-tetrahydropyranyl-cyclohexan (IIIa-i) wird dann mit primären Alkoholen wie z.B. Methanol, Ethanol in Gegenwart von sauren Katalysatoren wie z.B. anorganischen Säuren, Toluolsulfonsäure, Pyridinium-para-Toluolsulfonat, Amberlyst-H15 in cis-3S-Oxazyl-cyclohexan-1 R-ol (IVa-i) überführt, welches beispielsweise mit Bromomethylbenzoesäurealkylestern (VII-i mit Z4 = Br) unter basischen Bedingungen alkyliert (Verbindung der Formel (Ia-i) und schließlich zur Verbindung der Formel (I-i) hydrolysiert wird, sofern R3 = H ist. Falls im gewünschten Zielmolekül R3 = R6 = C₁-C₆-Alkyl oder Benzyl ist, braucht der Hydrolyseschritt nicht mehr durchgeführt werden, weil in diesem Fall die Verbindung (Ia-i) der Verbindung (I-i) entspricht. Die Hydrolyse kann im Sauren als auch im Basischen durchgeführt werden. Die basische Hydrolyse eignet sich bevorzugt für R6 = n-Alkyl, wobei mit Metallhydroxiden wie z.B. Alkali- oder Erdalkalihydroxiden in geeigneten Lösungsmitteln wie z.B. Wasser, Alkoholen, organischen Lösungsmitteln zu den gewünschten Stereoisomeren der PPAR-Aktivatoren verseift und die Carbonsäuregruppe durch Ansäuern freigesetzt wird. Wenn R6 = tert.-Butyl ist, wird die Hydrolyse bevorzugt im Sauren durchgeführt. Alternativ kann die Verbindung (I-i) auch durch Hydrogenolyse der Verbindung (lai) hergestellt werden. Dies bietet sich insbesondere an, sofern R6 = Benzyl ist und die entsprechende Verbindung (I-i) mit R3 = H gewünscht wird.

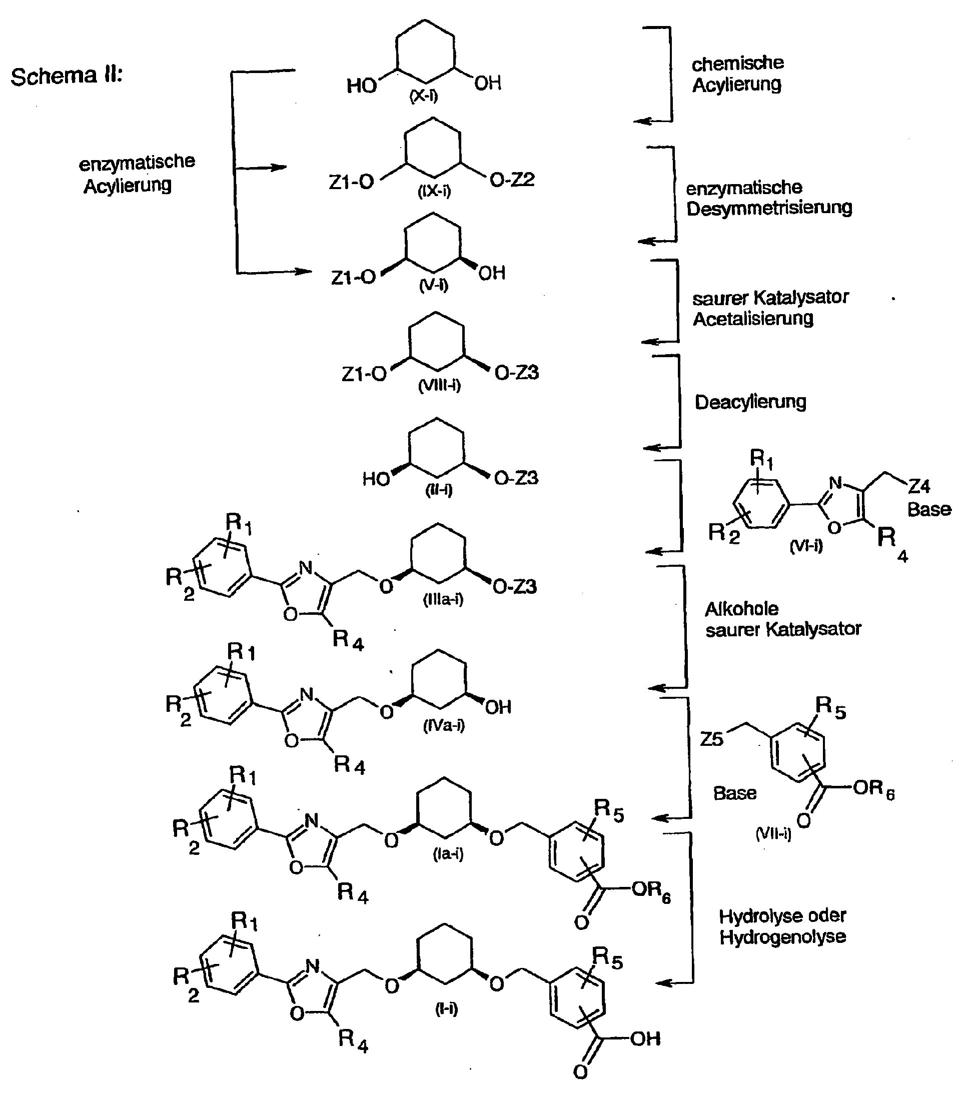

Wie aus den Ausführungen bezüglich des Schrittes a) des erfindungsgemäßen Verfahrens ersichtlich, kann die Verbindung der Formel (V) gemäß folgendem Verfahren erhalten werden, 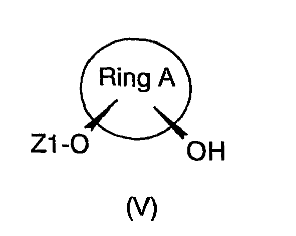 wobei
a1) eine Verbindung der Formel (IX) in Gegenwart der Lipase B aus Candida antarctica mit Wasser zu einer Verbindung der Formel (V) umgesetzt wird, 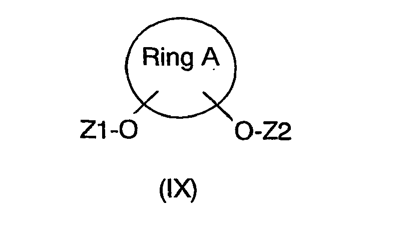
a2) eine Verbindung der Formel (X) in Gegenwart der Lipase B aus Candida antarctica mit mindestens einem Acyldonor zur Verbindung (V) umgesetzt wird, 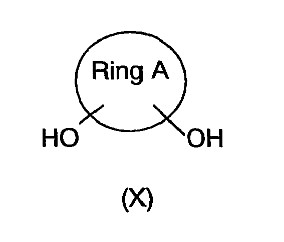 und die Verbindungen (IX) und (X) jeweils als reines cis-Isomer oder als cis/trans-Gemisch vorliegen, gegebenenfalls das trans-Isomer der Verbindung (IX) im Anschluss an Schritt a1) oder das trans-Isomer von Verbindung (X) im Anschluss an Schritt a2) abgetrennt wird,
   und worin die Variablen und Substituenten jeweils die nachfolgende Bedeutung haben:
   Ring A ist C₃-C₈-Cycloalkyl oder C₃-C₈-Cycloalkenyl, wobei in den Cycolalkyl-oder Cycloalkenylringen ein oder mehrere Kohlenstoffatome durch Sauerstoffatome ersetzt sein können,
   Z1 und Z2 sind unabhängig voneinander eine säurestabile Schutzgruppe.

Vorzugsweise sind in den Verbindungen (V) der Ring A gleich cis-1,3-Cyclohexyl, wobei das Kohlenstoffatom des Ringes A, das den OH-Substituenten aufweist, R-Konfiguration hat, und Z1 und Z2 gleich -C(O)-(C₁-C₃-Alkyl) sind.

Unter Bezugnahme auf die Ausführungen zu Schritt a) - wird nachfelgend ein Verfahren zur Auftrennung eines cis/trans-Gemisches einer Verbindung der Formel (X), beschrieben, beschrieben das dadurch gekennzeichnet ist dadurch, dass 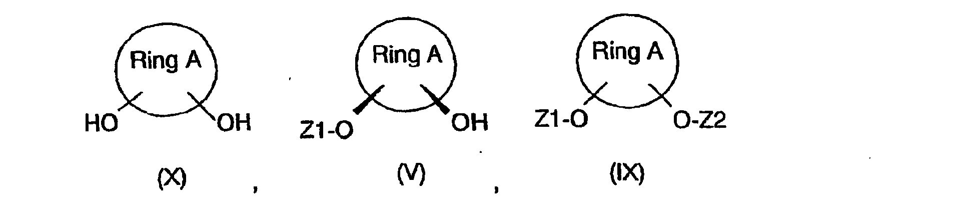
i) die Verbindung (X) mit mindestens einem Acyldonor zu einer Verbindung der Formel (IX) umgesetzt und die Verbindung (IX) mit Wasser zu einer Verbindung der Formel (V) in Gegenwart eines Enzyms, das einen geeigneten Enantiomerenüberschuß der Verbindung (V) liefert, umgesetzt wird und anschließend die dabei gebildete Verbindung (V) von dem nicht umgesetzten trans-Isomer der Verbindung (IX) und gegebenenfalls gebildeten sonstigen Nebenprodukten durch Chromatographie, Extraktion oder Destillation getrennt wird, oder
ii) die Verbindung (X) mit mindestens einem Acyldonor zu einer Verbindung (V) in Gegenwart eines Enzyms, das einen geeigneten Enantiomerenüberschuß der Verbindung (V) liefert, umgesetzt wird und anschließend die dabei gebildete Verbindung (V) von dem nicht umgesetzten trans-Isomeren der Verbindung (X) und gegebenenfalls gebildeten sonstigen Nebenprodukten durch Chromatographie, Extraktion oder Destillation getrennt wird, oder
iii) die Verbindung (X) mit mindestens einem Acyldonor zu einer Verbindung (IX) in Gegenwart eines Enzyms, das hauptsächlich das cis-Isomere der Verbindung (IX) liefert, umgesetzt wird und anschließend das cis-Isomere der Verbindung (IX) von dem ebenfalls gebildeten trans-Isomeren der Verbindung (V) und gegebenenfalls gebildeten sonstigen Nebenprodukten durch Chromatographie, Extraktion oder Destillation getrennt wird,
wobei die isolierten Fraktionen der Verbindungen (IX) und (V) in Gegenwart eines Nukleophils durch Abspaltung der Schutzgruppe Z1 und/oder Z2 gegebenenfalls in die entsprechende Verbindung (X) umgewandelt werden,
und worin die Variablen und Substituenten jeweils die nachfolgende Bedeutung haben:
Ring A ist C₃-C₈-Cycloalkyl oder C₃-C₈-Cycloalkenyl, wobei in den Cycolalkyl-oder Cycloalkenylringen ein oder mehrere Kohlenstoffatome durch Sauerstoffatome ersetzt sein können,
Z1 und Z2 sind unabhängig voneinander eine säurestabile Schutzgruppe, aus der Reihe -C(O)-C₁-C₆-Alkyl oder -C(O)-Phenyl.

Für die in den Verfahrensoptionen i) bis iii) erwähnten gegebenenfalls gebildeten sonstigen Nebenprodukte kommen - je nach der gewählten Verfahrensoption - beispielsweise das trans-Isomere der Verbindung (IX), das trans-Isomere der Verbindung (V) oder das cis-Isomere der Verbindung (IX) in Betracht. Diese je nach Reaktionsführung nur gegebenenfalls gebildeten sonstigen Nebenprodukte sowie eventuell noch vorhandenes Edukt können von den jeweils anfallenden Hauptfraktionen mit dem Fachmann bekannten Methode, eventuell in Form von zusätzlichen Chromatographie-, Extraktions- oder Destillationsschritten, abgetrennt werden.

Bevorzugt wird in diesem Trennverfahren die jeweilige Umsetzung nach i) und/oder ii) in Gegenwart der Lipase B aus Candida antarctica und/oder nach iii) in Gegenwart einer Lipase der Schweinepankreas, einer Lipase aus Burkholderia species, einer Upase aus Pseudomonas cepacia oder einer Lipase aus Pseudomonas species durchgeführt.

Besonders genannt sind in diesen Trennverfahren von cis/trans-Gemischen der Ring A gleich cis-1,3-Cyclohexyl, wobei das Kohlenstoffatom des Ringes A, das den OH-Substituenten aufweist, R-Konfiguration hat, und Z1 und Z2 gleich -C(O)-(C₁-C₃-Alkyl) sind.

Ein weiterer Gegenstand der vorliegenden Erfindung sind die in Schritt d) des erfindungsgemäßen Verfahrens als Zwischenprodukte erhältlichen Verbindungen der Formel (IIIa). 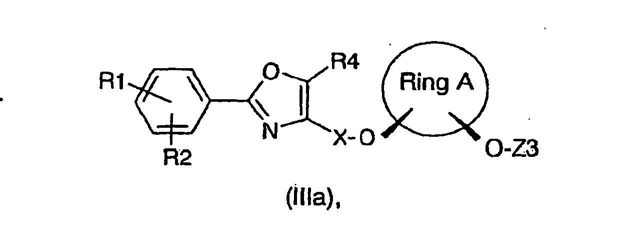 worin bedeuten:
R1, R2, R4 sind unabhängig voneinander H, F, Cl, Br, OH, NO₂, CF₃, OCF₃, C₁-C₆-Alkyl oder -O-(C₁-C₆-Alkyl),
X ist C₁-C₆-Alkyl, wobei in der Alkylgruppe ein oder mehrere Kohlenstoffatome durch Sauerstoffatome ersetzt sein können,
Ring A ist C₃-C₈-Cycloalkyl oder C₃-C₈-Cycloalkenyl, wobei in den Cycolalkyl-oder Cycloalkenylringen ein oder mehrere Kohlenstoffatome durch Sauerstoffatome ersetzt sein können,
Z3 ist eine basenstabile und säurelabile Acetal -oder Ketal- Schutzgruppe.
Bevorzugte Verbindungen der Formel (IIIa) weisen die folgenden Definitionen auf:
R1, R2, R4 sind unabhängig voneinander H, F, Cl, Br, OH, NO₂, CF₃, OCF₃, C₁-C₆-Alkyl oder -O-(C₁-C₆-Alkyl),
X ist C₁-C₃-Alkyl,
Ring A ist Cyclopentyl, Cyclohexyl oder Cycloheptyl,
Z3 ist eine basenstabile und säurelabile Acetal -oder Ketal- Schutzgruppe.

Mehr bevorzugte Verbindungen der Formel (IIIa) weisen die folgenden Definitionen auf:
Ring A ist Cyclohexyl, wobei der X-enthaltende und der Z3-enthaltende Substituent in cis-1,3-Position zum Cyclohexyl-Fragment stehen,
R1, R2 und R4 sind unabhängig voneinander H, F, Cl, C₁-C₃-Alkyl oder -O-(C₁-C₃-Alkyl),
Z3 ist Tetrahydropyranyl,
X ist Methyl.

Besonders bevorzugte Verbindungen der Formel (IIIa) weisen die folgenden Definitionen auf:
Ring A ist Cyclohexyl, wobei der X-enthaltende und der Z3-enthaltende Substituent in cis-1,3-Position zum Cyclohexyl-Fragment stehen und das Kohlenstoffatom des Ringes A, das mit -O-Z3 substituiert ist, R-Konfiguration aufweist,
R1, R2 und R4 sind unabhängig voneinander H, F, Cl, C₁-C₃-Alkyl oder -O-(C₁-C₃-Alkyl),
Z3 ist Tetrahydropyranyl,
X ist Methyl.

Ein weiterer Gegenstand der vorliegenden Erfindung sind die in Schritt d) des erfindungsgemäßen Verfahrens als Zwischenprodukte erhältlichen Verbindungen der Formel (IIIb). 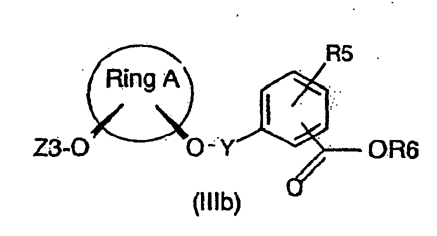 worin bedeuten:
R₅ ist unabhängig voneinander H, F, Cl, Br, OH, NO₂, CF₃, OCF₃, C₁-C₆-Alkyl oder -O-(C₁-C₆-Alkyl),
R6 ist C₁-C₆-Alkyl oder Benzyl, die gegebenenfalls mit F, Cl, Br, OH, NO₂, CF₃, OCF₃, C₁-C₆-Alkyl oder -O-(C₁-C₆-Alkyl) substituiert sein können,
Y ist C₁-C₆-Alkyl, wobei in der Alkylgruppe ein oder mehrere Kohlenstoffatome durch Sauerstoffatome ersetzt sein können,
Ring A ist C₃-C₈-Cycloalkyl oder C₃-C₈-Cycloalkenyl, wobei in den Cycolalkyl-oder Cycloalkenylringen ein oder mehrere Kohlenstoffatome durch Sauerstoffatome ersetzt sein können,
Z3 ist eine basenstabile und säurelabile Acetal -oder Ketal Schutzgruppe.

Bevorzugte Verbindungen der Formel (IIIb) weisen die folgenden Definitionen auf:
Ring A ist Cyclohexyl, wobei der X-enthaltende und der Z3-enthattende Substituent in cis-1,3-Position zum Cyclohexyl-Fragment stehen und das Kohlenstoffatom des Ringes A, das mit -O-Z3 substituiert substituiert ist, R-Konfiguration aufweist,
R5 ist H, F, Cl, C₁-C₃-Alkyl oder -O-(C₁-C₃-Alkyl),
R6 ist C₁-C₆-Alkyl oder Benzyl,
Z3 ist Tetrahydropyranyl,
Y ist Methyl.

Ein weiterer Gegenstand der vorliegenden Erfindung sind die in Schritt b) des erfindungsgemäßen Verfahrens als Zwischenprodukte erhältlichen Verbindungen der Formel (VIII). 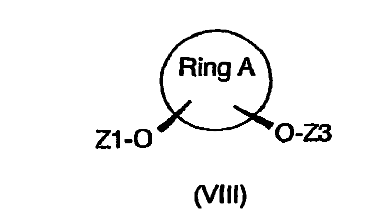 worin bedeuten:
Ring A ist Cyclohexyl, wobei der Z1-enthaltende und der Z3-enthaltende Substituent in cis-1,3-Position zum Cyclohexyl-Fragment stehen,
Z1 ist -C(O)CH₃,
Z3 ist Tetrahydropyranyl.

Besonders bevorzugte Verbindungen der Formel (VIII) weisen die folgenden Definitionen auf:
Ring A ist Cyclohexyl, wobei der Z1-enthaltende und der Z3-enthaltende Substituent in cis-1,3-Position zum Cyclohexyl-Fragment stehen und das Kohlenstoffatom des Ringes A, das mit -O-Z3 substituiert ist, R-Konfiguration aufweist,
Z1 ist -C(O)CH₃,
Z3 ist Tetrahydropyranyl.

Wie vorstehend beschrieben, können die Verbindungen der Formeln (IIIa) und (IIIb) gemäß der Schritte a) bis d) des erfindungsgemäßen Verfahrens hergestellt werden. Diesbezüglich gelten die gleichen Ausführungen wie für die Herstellung der Verbindungen der Formel (I). Entsprechendes gilt auch für die Verbindungen der Formel (VIII), die nach den Schritten a) bis b) des erfindungsgemäßen Verfahrens hergestellt werden können. Sämtliche dieser Zwischenverbindungen eignen sich folglich auch gegebenenfalls als Ausgangsverbindungen zur Synthese von PPAR-Aktivatoren der allgemeinen Formel (I).

Die nachfolgenden Beispiele sollen das Verfahren verdeutlichen, ohne es jedoch einzuschränken.

### Beispiele:

### GC-Analytik:

Bestimmung der Umsatzes: Supelco BetaDex325 (30 m x 0,25 mm x 0,25 µm), Trägergas H₂ (1,2 mL/min), Inj. 250° C, FID, Ofentemperatur 130°C isotherm, Split 100:1.

Retentionszeiten: 10,9 min cis-1-Acetoxy-cyclohexan-3-ol, 14,6 min cis-1,3-Diacetoxy-cyclohexan, 12,6 & 12,8 min *trans-*1,3-Diacetoxy-cyclohexan.

Zur Bestimmung des Enantiomerenüberschusses an *cis*-1S-Acetoxy-cyclohexa-3R-ol wird mit Heptafluorobuttersäureanhydrid derivatisiert: 2 mg der Probe werden in 0,1 mL Heptafluorobuttersäureanhydrid gelöst und die Reaktionsmischung 10 Minuten bei 70°C gehalten. Die Probe wird im Stickstoffstrom bis zur Trockene eingedampft und in 0,5 mL Methylenchlorid wieder aufgenommen. Retentionszeiten: 24,4 min, Derivat von *cis*-1S-Acetoxy-cyclohexa-3R-ol mit Heptafluorobuttersäure; 25,2 min, Derivat von *cis*-1R-Acetoxy-cyclohexa-3S-ol mit Heptafluorobuttersäure;

Supelco BetaDex325 (30 m x 0,25 mm x 0,25 µm), Trägergas: He (1,5 ml/min), Inj. 230° C, FID, Ofentemperatur 100°C isotherm, Split 50:1.

### Referenz- Beispiel 1: Umsetzung einer cis/trans-Mischung von Cyclohexan-1,3-diol mit Vinylacetat

4 mg einer 60 zu 40 Mischung von cis/trans-Cyclohexan-1,3-diol werden in 1 mL Vinylacetat gelöst und 5 mg Enzym gemäß Tabelle 1 zugegeben. Die Mischung wird bei 25°C temperiert und Proben nach 21 bzw. 45 h entnommen.

**Tabelle 1**

| **Handelsname** | **Organismus** | **Hersteller** | **NCBI Genbank Accession Nr. / Patentdokument** |
|---|---|---|---|
| Chirazyme L1 lyo. | Burkholderia cepacia | Roche Diagnostics | AAT85572 |
| Chirazyme L1 c.f. | Burkholderia cepacia | Roche Diagnostics | AAT85572 |
| Chirazyme L2 lyo. | Candida antarctica, Fraktion B | Roche Diagnostics | CAA83122; EP-A 287 634 |
| Chirazyme L2 c.f. C2 | Candida antarctica, Fraktion B | Roche Diagnostics | CAA83122 |
| Chirazyme L2 c.f. C3 | Candida antarctica, Fraktion B | Roche Diagnostics | CAA83122 |
| Chirazyme L7 lyo. | Schweinepankreas | Roche Diagnostics | P00591 |
| Lipase PS | Pseudomonas cepacia | Amano Enzymes | EP-A 331,376 |
| **Lipase AH** | **Pseudomonas sp.** | **Amano Enzymes** | |

Mit den Enzymen Chirazyme L2 lyo., Chirazyme L2 c.f. C2 und Chirazyme L2 c.f. C3 werden bevorzugt die cis-Monoacetate gebildet. Weiterhin katalysierten die Enzyme Chirazyme L7 lyo., Chirazyme L1 lyo., Chirazyme L1 c.f., Lipase PS und Lipase AH überraschenderweise bevorzugt die Bildung von cis-Diacetat aus cis-Cyclohexan-1,3-diol bei gleichzeitig hohen Umsatz, wohingegen die trans-Verbindung überwiegend nur zum trans-Monoacetat umgesetzt wird (Tabelle 2).

Die bei den jeweiligen Enzymen entstehenden Gemische aus Monoacetat und Diacetat können dann z.B. durch Extraktion oder Destillation getrennt werden. Dadurch gelingt auch die Abtrennung von cis-1,3-Diacetoxycyclohexan aus kommerziell erhältlichen cis/trans-Mischungen von Cyclohexan-1,3-diol.

**Tabelle 2**

| 1) Enzym | 2) Umsatz | 3) Monoacetat | 4) Diacetat | *5) Anteil an Cis-*Diacetat aus 4) |
|---|---|---|---|---|
| Chirazyme L7 lyo. | 94 % (45 h) | 34 % (trans) | 58,8 % | 98,2 % |
| Chirazyme L1 c.f. | 100 % (21 h) | 52,7 % (trans) | 47,3 % | 94,1 % |
| Lipase PS | 100 % (45 h) | 59,8 % (trans) | 40,2 % | 96,1 % |
| Lipase AH | 84 % (45 h) | 44,3 % (trans) | 55,7 % | 100 % cis |
| Chirazyme L2 c.f. C3 | 100 % (21 h) | 92,7 % (cis) | 7,3 % | 50 % |

### ReferenzBeispiel 2: Umsetzung einer cis/trans-Mischung von Cyclohexan-1,3-diol mit Vinylacetat zum 1,3-Diacetoxy-cyclohexan

30 g einer 60 zu 40 Mischung von cis/trans-Cyclohexan-1,3-diol werden in 900 mL Methyl-*tert*.-butylether gelöst, 100 ml Vinylacetat zugegeben und 1,7 g Lipase (Chira-zyme L1 lyo.) zugegeben (20°C). Nach 24 h wird das Protein abfiltriert und das Lösungsmittel entfernt. Das zurückbleibende Öl wird in 1000 ml Cyclohexan aufgenommen und viermal mit 150 mL Wasser gewaschen. Die organische Phase wird am Rotationsverdampfer entfernt. Man erhält 10g cis-1,3-Diacetoxy-cyclohexan als Öl (23 % Ausbeute bezogen auf das cis/trans Gemisch, 95 % *cis*).

### Referenz- Beispiel 3: Chemische Acylierung einer cis/trans-Mischung von Cyclohexan-1,3-diol zum 1,3-Diacetoxy-cyclohexan mit anschließender enzymatischer Desymmetrisierung zum cis-1 S-Acetoxy-cyclohexan-3R-ol

### Chemische Acylierung

cis/trans-Cyclohexan-1,3-diol wird durch einfache Acylierungsmethoden mit Acetanhydrid oder mit Acetylchlorid, wie sie z.B. aus dem Organikum Seite 405-7, 16.Auflage 1986 VEB Deutscher Verlag der Wissenschaften (Berlin), bekannt sind, zum cis/trans-1,3-Diacetoxy-Cyclohexan überführt.

### Enzymatische Desymmetrisierung

21 g einer 96 zu 4 Mischung cis/trans-1,3-Diacetoxy-Cyclohexan und 0,2 g Chirazyme L2 lyo. werden bei 25°C in 150 mL 0,1 M Kaliumphosphat-Puffer (pH 7,0) gegeben. Der pH-Wert wird durch Zugabe von 10 M KOH bei 7,0 gehalten und die Reaktionsmischung 5 Stunden gerührt. Die Reaktionsmischung wird dann dreimal mit je 100 mL Methylenchlorid extrahiert und das Lösungsmittel am Rotationsverdampfer entfernt. Man erhält 15,8 g *cis*-1S-Acetoxy-cyclohexan-3R-ol (Ausbeute 83 % bezogen auf das cis/trans Gemisch, ee > 99 %). Die Zuordnung der absoluten Konfiguration.erfolgte über die chirale HPLC-Analytik auf einer späteren Synthesestufe des cis-3S-(2-(4-Fluorphenyl)-oxa-zol-4-yl-methoxy)-cyclohexan-1 R-ol (Beispiel 8) durch Vergleich mit bekannten Referenzmaterial.

Das nachfolgende Schema III soll zur Verdeutlichung des erfindungsgemäßen Herstellverfahrens eines konkreten Beispiels (10) von Verbindungen der Formel (I) dienen.

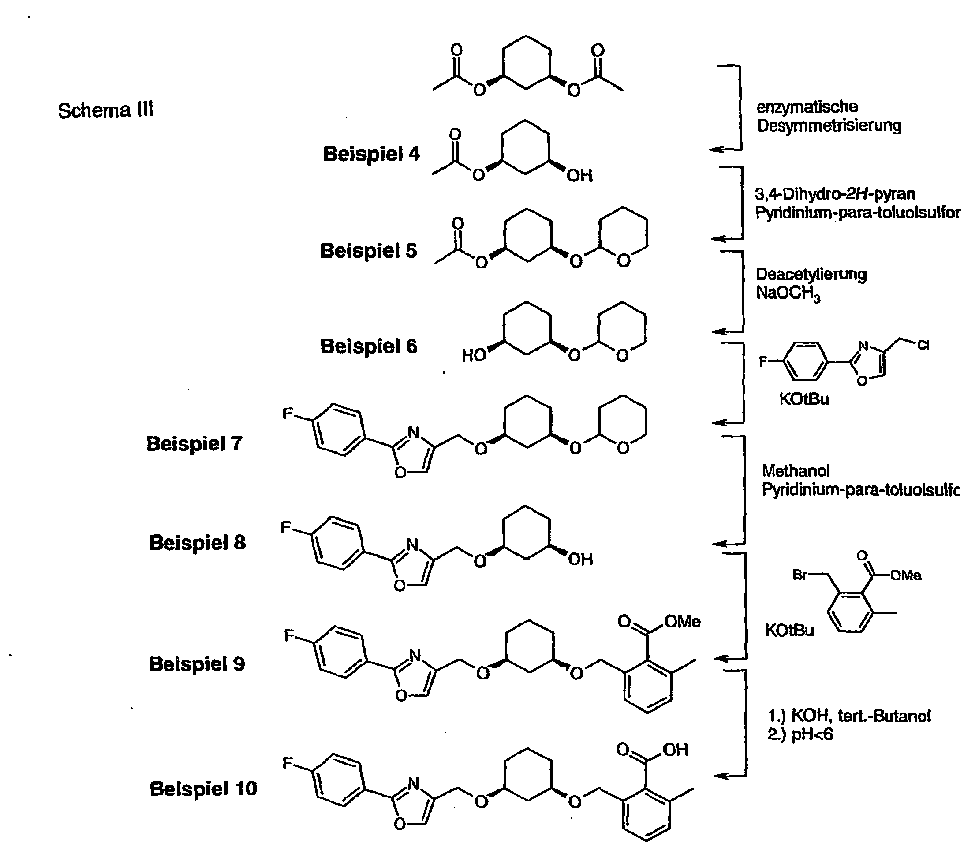

### Referenz Beispiel 4: Chemische Acylierung von cis-Cyclohexan-1,3-diol zum cis-1,3-Diacetoxy-cyclohexan mit anschließender enzymatische Desymmetrisierung zum cis-1S-Acetoxy-cyclohexan-3R-ol

### Chemische Acylierung

Cis-Cyclohexan-1,3-diol wird durch einfache Acylierungsmethoden mit Acetanhydrid oder mit Acetylchlorid, wie sie z.B. aus dem Organikum Seite 405-7, 16. Auflage 1986 VEB Deutscher Verlag der Wissenschaften (berlin), bekannt sind, zum cis-1,3-Diacetoxy-Cyclohexan überführt.

### Enzymatische Desymmetrisierung

166 g cis-1,3-Diacetoxy-cyclohexan und 1,6 g Chirazyme L2 lyo. werden in 1 L 0,1 M Kaliumphosphat-Puffer (pH 6,8) gegeben und der pH-Wert durch Zugabe von 10 M KOH konstant gehalten. Die Reaktionsmischung wird für 6 Stunden bei 35°C gehalten und dann auf 75°C für 1 Stunde erhitzt. Anschließend wird auf 8° C gekühlt und die Reaktionsmischung über Nacht stehengelassen. Das Protein wird abfiltriert und die Reaktionsmischung wird zweimal mit je 500 mL Toluol extrahiert. Das Lösungsmittel wird am Rotationsverdampfer entfernt. 121,3 g *cis*-1S-Acetoxy-cyclo-hexan-3R-ol werden als farbloses Öl erhalten (87 % Ausbeute, > 99 % ee).

### Beispiel 5: Einführung der Tetrahydropyranyl-(THP)-Schutzgruppe zu cis-1S-Acetoxy-3R-(O-tetrahydropyranyl)-cyclohexan

25 g 87 %iges (138 mmol) cis-1S-Acetoxy-cyclohexan-3R-ol (> 99 % ee) werden mit 15,6 g (166 mmol) 3,4-Dihydro-*2H*-pyran in Gegenwart von 1,76 g (0,05 Äq.) Pyridinium-para-toluolsulfonat in 125 ml Toluol eine Stunde unter Rühren auf 50 bis 60°C erhitzt. Die Reaktionskontrolle der quantitativen Umsetzung erfolgt mittels GC-Analyse. Das Reaktionsgemisch wird abgekühlt und das ausgefallene Pyridinium-para-toluolsulfonat abfiltriert. Die Entfernung des Pyridinium-para-toluolsulfonat durch eine Filtration ist nicht unbedingt nötig, da bei der anschließenden Deacetylierung überschüssig eingesetztes Natriummethanolat das saure Salz Pyridinium-para-toluolsulfonat neutralisiert und die Deacetylierung dadurch nicht beeinflusst wird. Das erhaltene Filtrat wird ohne weitere Reinigung in der anschließenden Deacetylierung zu cis-3R-(O-tetrahydropyranyl)-cyclohexan-1S-ol eingesetzt. Nach Einengen des Filtrates resultiert ein Öl, wobei als Hauptkomponente cis-1S-Acetoxy-3R-(O-tetra-hydropyranyl)-cyclohexan mit einem Molgewicht von 242,32 (C₁₃H₂₂O₄); MS (EI): 241 (M - H⁺) enthalten ist.

### Beispiel 6: Deacetylierung zu cis-3R-(O-tetrahydropyranyl)-cyclohexan-1Sol

Die toluolische Lösung von *cis*-1S-Acetoxy-3R-(O-tetrahydropyranyl)-cyclohexan aus Beispiel 5 wird mit 115 ml Methanol versetzt und mit 7,48 g (0,3 Äq.) 30 %ige Natriummethanolat-Methanol-Lösung zur Reaktion gebracht. Die quantitative Deacetylierung kann mittels GC-Analytik verfolgt werden. Die Aufarbeitung kann entweder nach Methode A) oder B) erfolgen:

### Methode A)

Nach ca. 2 Stunden Rühren bei Raumtemperatur wird die Reaktionsmischung mit 600 ml Wasser versetzt. Nach Abtrennung der wässrigen Phase wird die Toluolphase noch 3 mal mit jeweils 600 ml Wasser extrahiert. Die wässrigen Phasen werden vereinigt, mit NaCl gesättigt und dreimal mit jeweils 600 ml Toluol extrahiert. Die während des Verfahrens angefallenen übrigen cis-1,3-Cyclohexandiol-Derivate, d.h. diejenigen Ausgangsmaterialien, Nebenprodukte oder Vorstufen, die nicht Beispiel 6 oder dem mit 2 THP-Schutzgruppen gebildeten Nebenprodukt entsprechen, verbleiben als wasserlösliche Verbindungen in der wässrigen Phase. Die organischen Phasen werden vereinigt und unter Vakuum eingeengt. Cis-3-(O-tetrahydropyranyl)-cyclohexan-1S-ol wird als ein farbloses Öl mit einer Reinheit >97% und einer Ausbeute von >96% bezogen auf cis-1S-Acetoxy-cyclohexan-3R-ol erhalten. Das so hergestellte cis-3R-(O-tetrahydropyranyl)-cyclohexan-1S-ol hat ein Molekulargewicht von 200,28 (C₁₁H₂₀O₃); MS (EI): 199 (M - H⁺).

### Methode B)

Nach ca. 2 Stunden Rühren bei Raumtemperatur wird die Reaktionsmischung mit 200 ml Wasser versetzt und das zweiphasige Gemisch eine Stunde gerührt. Überschüssiges 3,4-Dihydro-2H-pyran, Methanol, Toluol und Wasser werden bei einer Innentemperatur bis 65°C bei 300 bis 150 mbar abdestilliert. Der viskose Rückstand wird mit 90 ml Toluol und 14 ml Wasser aufgenommen und mit 25 g Natriumchlorid versetzt. Nach Rühren dieses zweiphasigen Gemisches werden die Phasen getrennt und die wässrige Phase mit weiteren 30 ml Toluol extrahiert. Die während des Verfahrens angefallenen übrigen cis-1,3-Cyclohexandiol-Derivate, d.h. diejenigen Ausgangsmaterialien, Nebenprodukte oder Vorstufen, die nicht Beispiel 6 oder dem mit 2 THP-Schutzgruppen gebildeten Nebenprodukt entsprechen, verbleiben als wasserlösliche Verbindungen in der wässrigen Phase. Die organischen Phasen werden vereinigt und im Vakuum eingeengt. *Cis*-3-(O-tetra-hydro-pyranyl)-cyclohexan-1 S-ol wird als ein farbloses Öl mit einer Reinheit >95% und einer Ausbeute von >95% bezogen auf *cis*-1S-Acetoxy-cyclohexan-3R-ol erhalten. Das auf diese Weise hergestellte cis-3-(O-tetrahydro-pyranyl)-cyclohexan-1S-ol hat ein Molekulargewicht von 200,28 (C₁₁H₂₀O₃); MS (EI): 199 (M - H⁺).

### Beispiel 7: Alkylierung mit 4-(Chloromethyl)-2-(4-fluorophenyl)oxazol zu cis-1S-(2-(4-Fluor-phenyl)-oxazol-4-yl-methoxy)-3R-O-tetra-hydropyranyl-cyclohexan

1,5 g (7,2 mmol) cis-3R-(O-Tetrahydropyranyl)-cyclohexan-1S-ol werden in 40 ml Toluol mit 1,17 g (10,1 mmol) Kaliumtert.-butanolat unter Rühren 20 min auf 55°C erhitzt. Die Reaktionslösung wird dann tropfenweise bei 55°C eine toluolische Lösung von 1,74 g (7,9 mmol) 4-(Chloromethyl)-2-(4-fluorophehyl)oxazol in 26 ml Toluol zugegeben. Die Reaktionsmischung wird nach vollständiger Zugabe etwa 5 Stunden bei 55°C gerührt. Eine Reaktionskontrolle erfolgt per HPLC. Die Reaktionslösung wird anschließend mit 20 ml Wasser unter heftigem Rühren versetzt. Nach Einengen der vereinigten organischen Phasen resultiert ein braunes Öl mit einer 74%igen Reinheit von *cis*-1-(2-(4-Fluor-phenyl)-oxazol-4-yl-methoxy)-3-O-tetrahydropyranyl-cyclohexan mit einem Molekulargewicht von 375,44 (C₂₁H₂₆FNO₄); MS (EI): 375.

Die vereinigten organischen Phasen können auch ohne weitere Reinigung in der anschließenden Abspaltung der Tetrahydropyranyl-Schutzgruppe zu *cis*-3S-(2-(4-Fluor-phenyl)-oxazol-4-yl-methoxy)-cyclohexan-1 R-ol eingesetzt werden.

### Beispiel 8: Entfernung der Tetrahydropyranyl-Schutzgruppe zu cis-3S-(2-(4-Fluorphenyl)-oxazol-4-yl-methoxy)-cyclohexan-1R-ol

2,3 g (4,53 mmol) 74%iges *cis*-3S-(2-(4-Fluorphenyl)-oxazol-4-yl-methoxy)-cyclohex-an-1R-ol werden in 9 ml Toluol mit 3 ml Methanol in Anwesenheit von 113 mg (0,1 Äq.) Pyridinium-para-toluolsulfonat 6 Stunden bei 55°C gerührt. Anschließend wird die Reaktionslösung mit 10 ml ges. wässriger NaCl-Lösung extrahiert. Hierbei verbleibt in der wässrigen Phase gegebenenfalls vorhandenes 1,3-Cyclohexandiol, welches von dem in Beispiel 5 gegebenenfalls gebildeten Nebenprodukt stammt, das 2 THP-Schutzgruppen aufweist. Die organische Phase wird im Vakuum eingeengt. Das resultierende braune hochviskose Öl wird mit wenig Diisopropylether verrührt. Nach mehreren Stunden kristallisieren 44 % *cis-*3-(2-(4-Fluorphenyl)-oxazol-4-yl-methoxy)-cyclohexanol mit >99 %ee und einer Reinheit von 99%. Nach Einengen der Mutterlauge kristallisieren weitere 24% *cis-*3-(2-(4-Fluorphenyl)-oxazol-4-yl-methoxy)-cyclohexanol mit >99 % ee allerdings mit einer Reinheit von 89%. Durch weiteres Einengen der Mutterlaugen kann zwar noch weiteres *cis*-3S-(2-(4-Fluorphenyl)-oxazol-4-yl-methoxy)-cyclohexan-1R-ol kristallin erhalten werden, allerdings nehmen auch die Verunreinigungen mit jedem weiteren Einengen der Mutterlaugen in den kristallisierten Produkten zu. Eine einfache Chromatographie mit Essigsäureethylester/Toluol des oben als braunes Öl erhaltenen cis-3S-(2-(4-Fluorphenyl)-oxazol-4-yl-methoxy)-cyclohexan-1R-ol führt alternativ zu wesentlich besseren Ausbeuten. Nach Chromatographie und Einengen im Vakuum resultieren als weiße Kristalle cis-3S-(2-(4-Fluorphenyl)-oxazol-4-yl-methoxy)-cyclo-hexan-1R-ol mit > 99%ee. Das erhaltene cis-3S-(2-(4-Fluorphenyl)-oxazol-4-yl-me-thoxy)-cyclohexan-1R-ol hat ein Molekulargewicht von 291,32 (C₁₆H₁₈FNO₃); MS (TOF MS ES+): 291,9 (M+H⁺).

### Beispiel 9: Alkylierung mit 2-(Bromomethyl)-6-methylbenzoesäuremethyl-ester zu cis-2-(3S-(2-(4-Fluorphenyl)-oxazol-4-yl-methoxy)-cyclohexyl-1R-oxymethyl)-6-methylbenzoesäuremethylester

5 g (17,2 mmol) cis-3S-(2-(4-Fluorphenyl)-oxazol-4-yl-methoxy)-cyclohexan-1R-ol werden in 175 ml Chlorbenzol gelöst und bei 0°C mit 3,54 g (30,9 mmol) Kalium-tert.-butanolat versetzt. Alternativ können auch cis-3S-(2-(4-Fluorphenyl)-oxazol-4-yl-methoxy)-cyclohexan-1R-ol und Kalium-tert.-butanolat als Feststoffe vorgelegt und bei 0°C mit 175 ml Chlorbenzol versetzt werden. Innerhalb von 20 min werden unter Rühren bei 0°C 9,54 g (18,9 mmol) 2-Brommethyl-6-methyl-benzoesäuremethylester als 50%ige Cyclohexanlösung zugetropft. Nach Rühren bei 0°C über Nacht wird die Reaktionslösung dreimal mit je 40ml Wasser extrahiert. Die organische Phase wird über Magnesiumsulfat getrocknet und im Vakuum eingeengt. Es resultiert ein braunes Öl mit 80 bis 90% *cis*-2-(3S-(2-(4-Fluorphenyl)-oxazol-4-yl-methoxy)-cyclohexyl-1R-oxy-methyl)-6-methyl-benzoesäuremethylester, welches ohne weitere Reinigung in der nachfolgenden Verseifung eingesetzt werden kann. Der erhaltene *cis*-2-(3S-(2-(4-Fluorphenyl)-oxazol-4-yl-methoxy)-cyclohexyl-1 R-oxymethyl)-6-methyl-benzoesäuremethylester hat ein Molekulargewicht von 453,52 (C₂₆H₂₈FNO₅); MS (ESI): 454 (M+H⁺).

### Beispiel 10: Verseifung und Freisetzung durch Ansäuern der Carboxylfunktion zu cis-2-(3S-(2-(4-Fluorphenyl)-oxazol-4-ylmethoxy)-cyclohexyl-1R-oxy-methyl)-6-methyl-benzoesäure

### Beispiel 10 a)

7,79 g (15,1 mmol) 88 %iger *cis*-2-(3S-(2-(4-Fluorphenyl)-oxazol-4-yl-methoxy)-cyclohexyl-1R-oxy-methyl)-6-methyl-benzoesäuremethylester werden mit 2,99 g (45,3 mmol) 85 %igem Kaliumhydroxid in 78 ml tert.-Butanol bei 50°C über Nacht gerührt. Anschließend werden dreimal 78 ml Wasser zugegeben und jeweils im Vakuum ca. 80 ml Lösungsmittel abdestilliert, bis eine trübe hellgelbe Produktlösung resultiert. Diese Lösung wird dreimal mit jeweils 24 ml Methyl-tert.-butylether extrahiert. Nach Abtrennung der organischen Phase wird die wässrige Lösung mit 50 ml Aceton versetzt und mit 2M Salsäure auf einen pH-Wert 4-5 angesäuert. Die Lösung wird bei 0-5°C gerührt, wobei *cis*-2-(3S-(2-(4-Fluorphenyl)-oxazol-4-yl-methoxy)-cyclohexyl-1 R-oxy-methyl)-6-methyl-benzoesäure als weißer kristalliner Feststoff (Reinheit >95 %) gebildet wird, der durch eine Filtration abgetrennt werden kann. Die mit einer Ausbeute >80% als weißer kristalliner Feststoff erhaltene *cis*-2-(3S-(2-(4-Fluorphenyl)-oxazol-4-yl-methoxy)-cyclo-hexyl-1R-oxymethyl)-6-methyl-benzoesäure mit einem Enantiomerenüberschuß >99% und einer Reinheit >95% kann zur weiteren Reinigung aus Toluol kristallisiert werden. Anstelle von Toluol können für die Umkristallisation auch andere Lösungsmittel wie z.B. n-Butylacetat, Alkohole und Alkohol-WasserGemische eingesetzt werden.

Die erhaltene *cis*-2-(3S-(2-(4-Fluorphenyl)-oxazol-4-ylmethoxy)-cyclohexyl-1 R-oxy-methyl)-6-methyl-benzoesäure hat ein Molekulargewicht von 439,18 (C₂₅H₂₆FNO₅); MS (TOF MS ES+): 440,2 (M+H⁺).

### Beispiel 10 b)

11,88 g (18,9 mmol) 72 %iger *cis*-2-(3S-(2-(4-Fluorphenyl)-oxazol-4-yl-methoxy)-cyclohexyl-1R-oxy-methyl)-6-methyl-benzoesäuremethylester werden mit 6,23 g (94,4 mmol) 85%igem Kaliumhydroxid und 6 ml Wasser in 113 ml tert.-Butanol bei 70°C über Nacht gerührt. Anschließend werden dreimal 85 ml Wasser zugegeben und jeweils im Vakuum ca. 85 ml Lösungsmittel abdestilliert. Der Rückstand wird dreimal mit je 36 ml Methylisobutylketon extrahiert. Die vereinigten Wasserphasen werden mit 35 ml 2 M HCl auf pH 8 eingestellt und mit 21 ml n-Butylacetat versetzt. Mit weiteren 10,5 ml 2M HCl wird das 2-Phasengemisch auf pH 4 eingestellt und auf 90°C erhitzt. Die Phasen werden in einem vorgeheizten Scheidetrichter getrennt. Die organische Phase wird mit Aktivkohle auf 90°C erhitzt und filtriert. Das Filtrat wird auf Raumtemperatur abgekühlt, wobei cis-2-(3S-(2-(4-Fluorphenyl)-oxazol-4-yl-methoxy)-cyclohexyl-1R-oxy-methyl)-6-methylbenzoesäure als weißer Feststoff (Reinheit >90 %) kristallisiert. Die mit einer Ausbeute 75 % erhaltene *cis*-2-(3S-(2-(4-Fluor-phenyl)-oxazol-4-yl-methoxy)-cyclo-hexyl-1R-oxymethyl)-6-methylbenzoesäure mit einem Enantiomerenüberschuß >99% wird zur weiteren Reinigung aus n-Butylacetat kristallisiert, wobei eine Reinheit >97% mit einem Enantiomerenüberschuß >99% resultiert. Die erhaltene cis-2-(3S-(2-(4-Fluorphenyl)-oxazol-4-ylmethoxy)-cyclohexyl-1R-oxymethyl)-6-methyl-benzoesäure hat ein Molekulargewicht von 439,18 (C₂₅H₂₆FNO₅); MS (TOF MS ES+): 440,2 (M+H⁺).

Die nachfolgenden Beispiele 11 bis 14 zeigen die Synthese einer weiteren Verbindung der allgemeinen Formel (I) auf.

### Beispiel 11: Alkylierung mit 4-(Chloromethyl)-2-(3-methoxyphenyl)-5-methyloxazol zu cis-1S-(2-(3-Methoxyphenyl)-5-methyloxazol-4-yl-methoxy)-3R-O-tetrahydropyranylcyclohexan

Eine Suspension von 13,3 g (115 mmol) Kaliumtert.-butylat in 140 ml Toluol wird unter Rühren auf 55°C erwärmt. Eine Lösung von 8,58 g (40,2 mmol) cis-3R-(O-Tetrahydropyranyl)-cyclohexan-1 S-ol (Beispiel 6) in 88,5 ml Toluol wird unter Rühren 15 min bei 55°C zugetropft. Die Mischung wird nach vollständiger Zugabe 15 min bei 55°C nachgerührt. Dann werden 52,24 g (40 mmol) 4-(Chloromethyl)-2-(3-methoxyphenyl)-5-methyloxazol in Toluol (18,2 %ige Lösung) während 45 min bei 55°C zugetropft. Es wird bei 55°C eine Stunde nachgerührt. Anschließend wird das Reaktionsgemisch mit 60 ml Wasser und 30 ml gesättigter NatriumchloridLösung versetzt und unter Rühren auf Raumtemperatur abgekühlt. Die Phasen werden getrennt und die organische Phase am Rotationsverdampfer eingeengt. Der ölige Rückstand wird durch eine Kieselgelchromatographie mit Toluol/Essigsäureethylester 3:1 gereinigt. Nach Einengen im Vakuum resultiert ein Öl von *cis*-1-(2-(3-Methoxyphenyl)-5-methyloxazol-4-yl-methoxy)-3-O-tetrahydro-pyranylcyclohexan mit einem Molekulargewicht von 401,5 (C₂₃H₃₁NO₅); MS (EI): 401 (M⁺).

Die toluolische Lösung von *cis*-1-(2-(3-Methoxy-phenyl)-5-methyl-oxazol-4-yl-methoxy)-3-O-tetrahydropyranyl-cyclohexan kann auch ohne weitere Reinigung in der anschließenden Abspaltung der Tetrahydropyranyl-Schutzgruppe zu cis-3S-(2-(3-methoxyphenyl)-5-methyl-oxazol-4-yl-methoxy)-cyclohexan-1R-ol eingesetzt werden, da ein Lösungsmittelwechsel nicht notwendig ist

### Beispiel 12: Entfernung der Tetrahydropyranyl-Schutzgruppe zu cis-3S-(2-(3-Methoxyphenyl)-5-methyl-oxazol-4-yl-methoxy)-cyclohexan-1R-ol

8,04 g (20 mmol) *cis*-1-(2-(3-Methoxy-phenyl)-5-methyl-oxazol-4-yl-methoxy)-3-O-te-trahydropyranyl-cyclohexan in Toluol wird mit 13 ml Methanol und 0,3 ml 30%ige Salzsäure versetzt und anschließend ca. 2 Stunden auf 55°C erhitzt. Das Reaktionsgemisch wird mit 25 ml Wasser versetzt. Nach der Phasentrennung wird die organische Phase bei 40°C im Vakuum eingeengt. Es resultieren 6,03 g (Ausbeute 95%) braunes Öl, welches in 10 ml Toluol gelöst und mittels Säulenchromatographie mit Toluol/Essigsäureethylester 1:2 gereinigt wird. Nach Entfernen der organischen Lösungsmittel erhält man 3,25 g (69,8 %) cis-3S-(2-(3-Methoxyphenyl)-5-methyl-oxazol-4-yl-methoxy)-cyclohexan-1 R-ol als braunes, viskoses Öl mit dem Molekulargewicht von 317,39 (C₁₈H₂₃NO₄); MS (EI): 317 (M⁺).

### Beispiel 13: Alkylierung mit 2-(Bromomethyl)-6-methyl-benzoesäuremethyl-ester zu cis-2-(3S-(2-(3-Methoxyphenyl)-5-methyloxazol-4-yl-methoxy)-cyclohexyl-1R-oxymethyl)-6-methylbenzoesäuremethylester

59 g (89,3 mmol) *cis*-3S-(2-(3-Methoxyphenyl)-5-methyloxazol-4-yl-methoxy)-cyclohexan-1R-ol werden in 825 ml Chlorbenzol gelöst und bei 0°C mit 17,7 g (158 mmol) Kalium-tert.-butanolat versetzt. Innerhalb von 20 min werden unter Rühren bei 0°C 9,54 g (18,9 mmol) 2-Brommethyl-6-methyl-benzoesäuremethylester als 50%ige Cyclohexanlösung zugetropft. Nach Rühren über Nacht bei 0°C wird die Reaktionslösung mit 350 ml ges. NaCl-Lösung versetzt und gerührt. Die organische Phase wird abgetrennt und im Vakuum eingeengt. Es resultiert ein braunes Öl mit 80% *cis*-2-(3S-(2-(3-Methoxyphenyl)-5-methyloxazol-4-yl-methoxy)-cyclohexyl-1R-oxymethyl)-6-methylbenzoesäure-methylester, welches ohne weitere Reinigung in der nachfolgenden Verseifung eingesetzt oder durch eine Kieselgelchromatographie mit Heptan/Essigsäureethylester vor der weiteren Umsetzung gereinigt werden kann. Der mit 64% Ausbeute erhaltene cis-2-(3S-(2-(3-Methoxyphenyl)-5-methyloxazol-4-yl-methoxy)-cyclohexyl-1R-oxymethyl)-6-methylbenzoesäuremethylester hat ein Molekulargewicht von 479,58 (C₂₈H₃₃NO₆); MS (TOF MS ES+): 480,2 (M+H⁺).

### Beispiel 14: Verseifung und Freisetzung durch Ansäuern der Carboxylfunktion zu cis-2-(3S-(2-(3-Methoxyphenyl)-5-methyl-oxazol-4-ylmethoxy)-cyclohexyl-1R-oxy-methyl)-6-methylbenzoesäure

### Beispiel 14 a)

7 g (14,6 mmol) 96,5 %iges *cis*-2-(3S-(2-(3-Methoxyphenyl)-5-methyloxazol-4-yl-methoxy)-cyclohexyl-1R-oxy-methyl)-6-methylbenzoesäuremethylester werden mit 7 ml 33%iger Natronlauge in 70 ml Ethanol auf Rückfluss 25 h erhitzt. Das Reaktionsgemisch wird im Vakuum vollständig eingeengt. Der ölige Rückstand wird mit 71 ml Wasser gelöst und anschließend zweimal mit je 35 ml Methyl-tert.-butylether extrahiert. Die wässrige Produktphase wird mit 70 ml Dichlormethan versetzt und 5 min gerührt. Mit 11,9 ml 30%iger Salzsäure wird das 2-Phasengemisch auf pH 1 gestellt. Die Phasen werden getrennt und die Wasserphase nochmals mit Dichlormethan extrahiert. Die gesammelten organischen Phasen werden über Magnesiumsulfat getrocknet und im Vakuum eingeengt. Das zurückbleibende hochviskose Öl wird mit 50 ml Diisopropylether bei 35°C gelöst. Nach Animpfen der Lösung mit 10 mg *cis*-2-(3S-(2-(3-Methoxyphenyl)-5-methyloxazol-4-yl-methoxy)-cyclohexyl-1R-oxy-methyl)-6-methylbenzoesäure setzt nach 30 min Kristallisation ein, die durch mehrstündiges Kühlen auf 0-5°C verbessert werden kann. Die Kristalle werden abgesaugt und im Vakuum bei 40°C getrocknet .Die mit einer Ausbeute von 72,1% erhaltene cis-2-(3S-(2-(3-Methoxy-phenyl)-5-methyl-oxazol-4-yl-methoxy)-cyclohexyl-1R-oxymethyl)-6-methylbenzoesäure mit einem Enantiomerenüberschuß >99% und einer Reinheit >99% kann zur weiteren Reinigung aus n-Butylacetat umkristallisiert werden.

Die erhaltene *cis*-2-(3S-(2-(3-Methoxyphenyl)-5-methyl-oxazol-4-ylmethoxy)-cyclohexyl-1R-oxy-methyl)-6-methylbenzoesäure hat ein Molekulargewicht von 465,55 (C₂₇H₃₀NO₆); MS (ES+): 466,3 (M+H⁺).

### Beispiel 14 b)

Die Verseifung von 7,9 g (16,5 mmol) *cis*-2-(3S-(2-(3-Methoxyphenyl)-5-methyloxazol-4-yl-methoxy)-cyclohexyl-1 R-oxy-methyl)-6-methylbenzoesäuremethylester erfolgt direkt nach der Alkylierung von *cis*-3S-(2-(3-Methoxyphenyl)-5-methyloxazol-4-yl-methoxy)-cyclohexan-1R-ol mit 2-Brommethyl-6-methylbenzoesäuremethylester in Anwesenheit von Kalium-tert.-butanolat, wie in Beispiel 13 beschrieben, in 750 ml Chlorbenzol ohne wässrige Aufarbeitung durch Zugabe von 7,62 g (115 mmol) 85%iger KOH zum Reaktionsgemisch und Erhitzen auf 80°C innerhalb von 3 Stunden. Das Reaktionsgemisch wird nach Abkühlen auf Raumtemperatur mit 30 ml Wasser extrahiert. Die Chlorbenzollösung wird anschließend nochmals mit 80 ml Wasser extrahiert. Die vereinigten Wasserphasen werden mit 40 ml Methylenchlorid versetzt und mit 10 ml 2M HCl auf pH 2 eingestellt. Nach kräftigem Rühren werden die Phasen getrennt und die organische Phase im Vakuum eingeengt. Der Rückstand wird mit Diisopropylether aufgenommen und wie oben in Beispiel 14 a analog aufgearbeitet.

Die nachfolgenden Beispiele 15 bis 19 sowie Schema IV zeigen die Synthese einer weiteren Verbindung der allgemeinen Formel (I) auf.

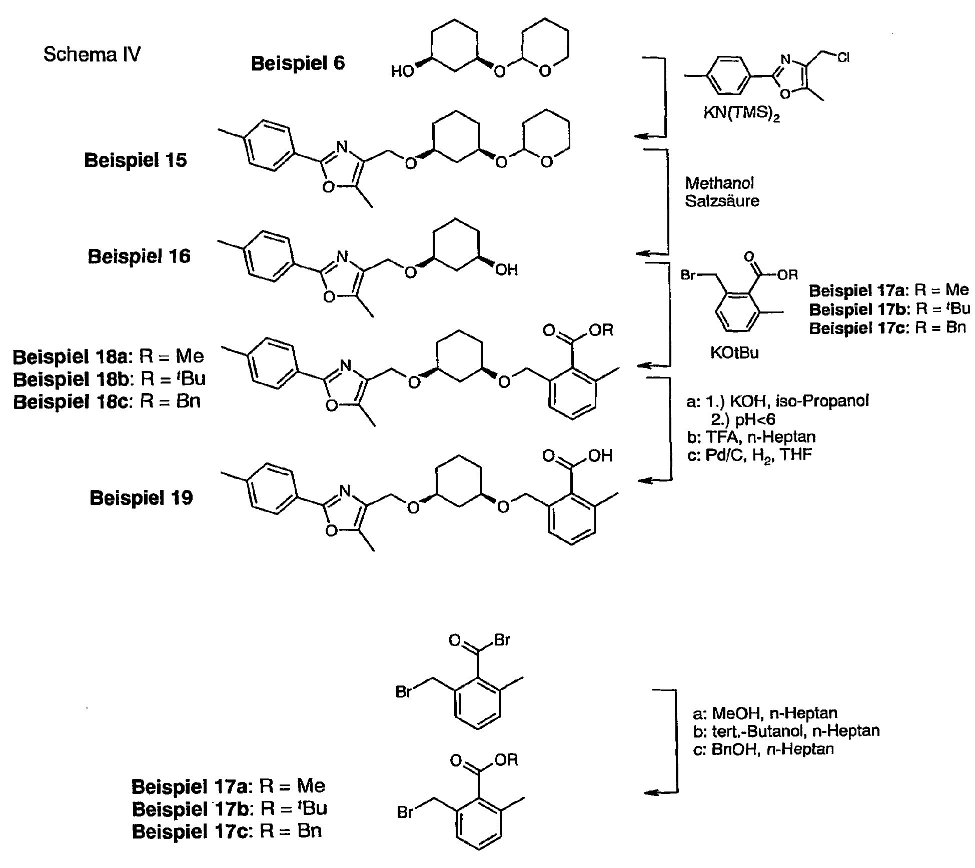

In dieser weiteren Beispielsynthese wird Beispiel 6 mit 4-(Chloromethyl)-5-methyl-2-p-tolyl-oxazol in Gegenwart von beispielsweise Alkali- und Erdalkalibasen, bevorzugt in Gegenwart von Kalium-tert.-butylat oder Kalium-bis-(trimethylsilyl)-amid, zu Beispiel 15 umgesetzt. Anschließend wird Beispiel 15 mit Methanol in Gegenwart von sauren Katalysatoren wie z.B. anorganischen Säuren, Toluolsulfonsäure, Pyridinium-para-Toluolsulfonat, Amberlyst-H15, bevorzugt mit anorganischen Säuren in Beispiel 16 überführt. 2-Bromomethyl-6-methylbenzoylbromid reagiert mit Alkoholen wie Methanol (Me-OH), tert.-Butanol (tBu-OH) oder Benzylalkohol (Bn-OH) zu Beispiel 17a, Beispiel 17b bzw. Beispiel 17c. Beispiel 16 wird mit Beispiel 17a, Beispiel 17b bzw. Beispiel 17c in Gegenwart von beispielsweise Alkali- und Erdalkalibasen, bevorzugt in Gegenwart von Kalium-tert.-butylat, zu den Beispiel 18a, Beispiel 18b bzw. Beispiel 18c alkyliert. Beispiel 18a wird mit Metallhydroxiden wie z.B. Alkali- und Erdalkalihydroxiden, bevorzugt Natrium- und Kaliumhydroxide, in geeigneten Lösungsmittel wie Wasser, Alkoholen oder organischen Lösungsmitteln, bevorzugt in Ethanol oder Isopropanol, verseift. Nach Ansäuern z.B. mit organischen oder anorganischen Säuren, bevorzugt mit anorganischen Säuren, wird der gewünschte chirale PPAR-Aktivator Beispiel 19 isoliert. Beispiel 19 wird ebenfalls durch Esterspaltung mit sauren Katalysatoren wie z.B. anorganischen Säuren oder Trifluoressigsäure (TFA), bevorzugt Salzsäure oder Trifluoressigsäure, aus Beispiel 18b erhalten. Beispiel 18c wird durch Hydrogenolyse mit einem heterogenen Katalysator, bevorzugt einem Edelmetallkatalysator und sehr bevorzugt einem Palladiumkatalysator in Beispiel 19 überführt. Beispiel 19 wird in einer optischen Reinheit von >99% ee isoliert, wobei weder eine chirale noch eine achirale Chromatographie zur Aufreinigung benötigt wird. Die Aufreinigung wird durch Kristallisation von Beispiel 16 und des PPAR-Aktivators Beispiel 19 z.B. aus einem organischen Lösungsmittel, bevorzugt aus Diisopropylether, durchgeführt.

### Beispiel 15: Alkylierung von cis-3R-(O-Tetrahydropyranyl)-cyclohexan-1S-ol mit 4-(Chloromethyl)-5-methyl-2-p-tolyl-oxazol zu cis-1S-(2-p-tolyl-5-methyl-oxazol-4-yl-methoxy)-3R-O-tetrahydropyranyl-cyclohexan

Zu einer Suspension von 130 g (652 mmol) Kalium-bis-(trimethylsilyl)-amid in 1,5 L tert.-Butylmethylether wird unter Rühren eine Lösung von 110 g (550 mmol) cis-3R-(O-tetrahydropyranyl)-cyclohexan-1S-ol (Beispiel 6) in 500 mL tert.-Butylmethylether und 97 ml Toluol gegeben. Das Reaktionsgemisch wird 10 Minuten bei Raumtemperatur gerührt, bevor eine Lösung von 111 g (501 mmol) 4-(Chloromethyl)-5-methyl-2-p-tolyl-oxazol in 1,5 L tert.-Butylmethylether zugegeben wird. Die Reaktion wird bis zum vollständigen Umsatz (HPLC) bei 35°C gerührt. Ein Aliquot des Reaktionsgemisches wird mit Wasser gewaschen, über Magnesiumsulfat getrocknet und anschließend im Vakuum vollständig eingeengt. Das auf diese Weise hergestellte *cis-*1 S-(2-p-Tolyl-5-methyloxazol-4-yl-methoxy)-3R-O-tetrahydropyranylcyclohexan hat ein Molekulargewicht von 385,51 (C₂₃H₃₁NO₄) MS (ESI): 302 [M - THP + H]⁺.

### Beispiel 16: Abspaltung der THP-Schutzgruppe von cis-3S-(2-p-Tolyl-5-methyl-oxazol-4-yl-methoxy)-3R-O-tetrahydropyranylcyclohexan zu cis-3S-(2-p-Tolyl-5-methyl-oxazol-4-yl-methoxy)-cyclohexan-1R-ol

Zu der Lösung von *cis*-3S-(2-p-Tolyl-5-methyl-oxazol-4-yl-methoxy)-3R-O-tetrahydropyranylcyclohexan in tert.-Butylmethylether und Toluol aus Beispiel 15 wird 100 mL Methanol und anschließend 101 mL konz. Salzsäure zugegeben. Die Reaktion wird bei 25°C gerührt. Bei vollständigem Umsatz wird 1.0 L Wasser und 101 g (1.2 mol) Natriumhydrogencarbonat zugegeben und das Gemisch intensiv gerührt. Nach Filtration wird die wässrige Phase abgetrennt und die organische Phase dreimal mit je 800 mL Wasser gewaschen. Das Lösungsmittel wird im Vakuum abdestilliert und der resultierende ölige Rückstand in 450 mL Diisopropylether aufgenommen. Das Gemisch wird bei Raumtemperatur gerührt. Das ausgefallene Produkt wird abfiltriert und mit wenig Diisopropylether gewaschen. Man erhält 56% *cis*-3S-(2-p-Tolyl-5-methyl-oxazol-4-yl-methoxy)-cyclohexan-1R-ol mit einem Molekulargewicht von 301,39 (C₁₈H₂₃NO₃); MS (ESI): 302 [M + H]⁺.

### Beispiel 17a: Synthese von 2-Bromomethyl-6-methyl-benzoesäuremethylester aus 2-Bromomethyl-6-methyl-benzoylbromid

Zu 400 mL Methanol werden 200 g (240 mmol) 2-Bromomethyl-6-methyl-benzoyl-bromid-Lösung (35%ige Lösung in Heptan) zugegeben. Nach einstündigem Rühren bei RT werden 400 mL n-Heptan zugegeben. Die Lösung wird nacheinander mit 600 mL ges. Natriumhydrogencarbonatlösung und dreimal mit je 200 mL Wasser gewaschen. Anschließend wird das Lösungsmittel im Vakuum vollständig entfernt. Man erhält 98.8% 2-Bromomethyl-6-methylbenzoesäuremethylester als farbloses Öl mit einer Reinheit von 80% (in Ausbeute eingerechnet) und mit einem Molekulargewicht von 241,99 (C₁₀H₁₁BrO₂); MS (ESI): 243,1 [M + H]⁺.

### Beispiel 17b: Synthese von 2-Bromomethyl-6-methyl-benzoesäure-tert.-butylester

Zu einem Gemisch aus 15 mL n-Heptan und 25 ml tert.-Butanol werden 20 g (24 mmol) 2-Bromomethyl-6-methyl-benzoylbromid-Lösung (35%ige Lösung in Heptan) zugegeben. Nach einstündigem Rühren bei 40°C werden 400 mL n-Heptan zugegeben. Die Lösung wird nacheinander mit 60 mL ges. Natriumhydrogencarbonatlösung und viermal mit je 20 mL Wasser gewaschen. Anschließend wird das Lösungsmittel im Vakuum vollständig entfernt. Bei quantitativen Umsatz erhält man 2-Bromomethyl-6-methylbenzoesäure-tert.-butylester als beinahe farbloses Öl mit 93%iger Reinheit und mit einem Molekulargewicht von 284,04 (C₁₃H₁₇BrO₂), MS (ESI): 302,1 [M + NH₄]⁺.

### Beispiel 17c: Synthese von 2-Bromomethyl-6-methyl-benzoesäurebenzylester

Zu 136 g (233 mmol) 2-Bromomethyl-6-methylbenzoylbromid-Lösung (35%ige Lösung in Heptan) gibt man unter Eiskühlung 24,2 g (221 mmol) Benzylalkohol zu. Anschließend lässt man eine Stunde bei RT rühren. Nach Zugabe von 500 mL tert.-Butylmethylether wird die organische Lösung nacheinander mit 410 mL ges. Natriumhydrogencarbonatlösung und viermal mit je 300 mL Wasser gewaschen. Anschließend wird das Lösungsmittel im Vakuum vollständig entfernt. Man erhält 74 g 2-Bromomethyl-6-methylbenzoesäurebenzylester als farbloses Öl mit einer Reinheit von 80%. Der auf diese Weise hergestellt 2-Bromomethyl-6-methyl-benzoesäurebenzylester hat ein Molekulargewicht von 318.02 (C₁₆H₁₅BrO₂); MS (ESI): 341,0 [M + Na]⁺

### Beispiel 18a: Alkylierung von cis-3S-(2-p-Tolyl-5-methyl-oxazol-4-yl-methoxy)-cyclohexan-1R-ol mit 2-Bromomethyl-6-methyl-benzoesäuremethylester zu 2-Methyl-6-[(1R,3S)-3-(5-methyl-2-p-tolyl-oxazol-4-yl-methoxy)-cyclohexyloxymethyl]-benzoesäuremethylester

50 g (166 mmol) *cis*-3S-(2-p-Tolyl-5-methyl-oxazol-4-yl-methoxy)-cyclohexan-1R-ol und 30 g (262 mmol) Kalium-tert.-butylat werden in 500 mL THF gelöst und das Gemisch wird 30 Minuten bei RT gerührt. Nach Zugabe von 500 mL tert.-Butylmethylether wird eine Lösung von 51 g (210 mmol) 2-Bromomethyl-6-methyl-benzoesäuremethylester in 310 mL tert.-Butylmethylether zugegeben. Die Reaktion wird 1 Stunde bei Temperaturen zwischen 25°C und 30°C gerührt und wird durch Zugabe von 500 mL Salzsäure (0,5 molar) gequencht. Nach Abtrennung der wässrigen Phase wird die organische Phase dreimal mit je 300 mL Wasser gewaschen. Anschließend wird das Lösungsmittel im Vakuum vollständig entfernt. Man erhält 74 g 2-Methyl-6-[(1 R,3S)-3-(5-methyl-2-p-tolyl-oxazol-4-yl-methoxy)-cyclohexyloxymethyl]-benzoesäuremethylester als leicht gelbes Öl mit einer Reinheit von 79% und mit einem Molekulargewicht von 463,57 (C₂₈H₃₃NO₅); MS (ESI): 464,37 [M + H]⁺.

### Beispiel 18b: Alkylierung von cis-3S-(2-p-Tolyl-5-methyl-oxazol-4-yl-methoxy)-cyclohexan-1R-ol mit 2-Bromomethyl-6-methyl-benzoesäure-tert.-butylester zu 2-Methyl-6-[(1R,3S)-3-(5-methyl-2-p-tolyl-oxazol-4-yl-methoxy)-cyclohexyloxymethyl]benzoesäure-tert.-butylester

100 g (332 mmol) cis-3S-(2-p-Tolyl-5-methyl-oxazol-4-yl-methoxy)-cyclohexan-1R-ol und 60 g (524 mmol) Kalium-tert.-butylat werden in 1,0 L THF gelöst und das Gemisch wird 30 Minuten bei RT gerührt. Nach Zugabe von 1 L tert.-Butylmethylether wird eine Lösung von 120 g (524 mmol) 2-Bromomethyl-6-methylbenzoesäuremethylester in 850 mL tert.-Butylmethylether zugegeben. Die Reaktion wird 30 Minuten bei Temperaturen bei 40°C gerührt. Anschließend wird die organische Phase nacheinander mit 1,0 L Wasser, 250 mL Salzsäure (0,5 molar) und viermal mit je 500 mL wässriger Natriumchloridlösung (1%ig) gewaschen. Nach vollständigem Entfernen des Lösungsmittels im Vakuum erhält man 195 g 2-Methyl-6-[(1 R,3S)-3-(5-methyl-2-p-tolyloxazol-4-yl-methoxy)-cyclohexyloxymethyl]-benzoesäure-tert.-butylester als gelbes Öl mit einer Reinheit von 86%. Der auf diese Weise hergestellt 2-Methyl-6-[(1 R,3S)-3-(5-methyl-2-p-tolyloxazol-4-yl-methoxy)-cyclohexyloxymethyl]-benzoesäure-tert.-butylester hat ein Molekulargewicht von 505,65 (C₃₁H₃₉NO₅); MS (ESI): 506,39 [M + H]⁺.

### Beispiel 18c: Alkylierung von cis-3S-(2-p-Tolyl-5-methyl-oxazol-4-yl-methoxy)-cyclohexan-1R-ol mit 2-Bromomethyl-6-methyl-benzoesäurebenzylester zu 2-Methyl-6-[(1R,3S)-3-(5-methyl-2-p-tolyl-oxazol-4-yl-methoxy)-cyclohexyloxymethyl]-benzoesäurebenzylester

Zu einer Lösung von 30 g (100 mmol) *cis*-3S-(2-p-Tolyl-5-methyl-oxazol-4-yl-methoxy)-cyclohexan-1R-ol in 300 mL THF werden 18 g (157 mmol) Kalium-tert.-butylat gegeben und das Gemisch wird 30 Minuten bei RT gerührt. Nach Zugabe von 44,5 g (112 mmol) 2-Bromomethyl-6-methylbenzoesäurebenzylester (80%ig) wird die Reaktion 30 Minuten bei 30°C gerührt. Anschließend wird die Reaktion auf eine Lösung von 15 mL Salzsäure (32%ig) in 300 mL Wasser gegossen. Nach Phasentrennung wird die organische Phase im Vakuum vollständig eingeengt und der Rückstand in 500 mL tert.-Butylmethylether aufgenommen. Die Lösung wird viermal mit je 300 mL Wasser gewaschen. Das Lösungsmittel wird im Vakuum entfernt und der Rückstand durch Chromatographie an Kieselgel gereinigt. In 90% Ausbeute erhält man Methyl-6-[(1R,3S)-3-(5-methyl-2-p-tolyl-oxazol-4-yl-methoxy)-cyclohexyloxymethyl]-benzoesäurebenzylester als gelbes Öl mit einem Molekulargewicht von 539.68 (C₃₄H₃₇NO₅); MS (ESI): 540,41 [M +H]⁺.

### Beispiel 19: Synthese von 2-Methyl-6-[(1R,3S)-3-(5-methyl-2-p-tolyl-oxazol-4-ylmethoxy)-cyclohexyloxymethyl]-benzoesäure durch Ester-spaltung

a: aus Methyl-6-[(1R,3S)-3-(5-methyl-2-p-tolyl-oxazol-4-yl-methoxy)-cyclohexyloxymethyl]-benzoesäuremethylester: 97,1 g (165 mmol) Methyl-6-[(1 R,3S)-3-(5-methyl-2-p-tolyl-oxazol-4-yl-methoxy)-cyclohexyloxymethyl]-benzoesäuremethylester werden in einem Gemisch aus 640 mL Isopropanol und 80 mL Wasser gelöst. 47,7 g (809 mmol) Kaliumhydroxid werden portionsweise zugegeben. Die Reaktion wird 24 Stunden bei 95°C gerührt. Anschließend wird das Lösungsmittel im Vakuum vollständig entfernt und der Rückstand in 800 mL tert.-Butylmethylether und 800 mL Wasser aufgenommen. Durch Zugabe von Salzsäure (2 M) wird das Gemisch angesäuert. Nach Phasentrennung wird die organische Phase viermal mit je 200 mL Wasser gewaschen. Die organische Phase wird im Vakuum eingeengt und der Rückstand mit wenig Diisopropylether verrührt. Der Feststoff wird filtriert und im Vakuum getrocknet. Man erhält 2-Methyl-6-[(1R,3S)-3-(5-methyl-2-p-tolyl-oxazol-4-ylmethoxy)-cyclohexyloxymethyl]-benzoesäure in 57% Ausbeute als kristallinen Feststoff.
b: aus Methyl-6-[(1R,3S)-3-(5-methyl-2-p-tolyl-oxazol-4-yl-methoxy)-cyclohexyloxymethyl]-benzoesäure-tert.-butylester: 200 g (396 mmol) Methyl-6-[(1R,3S)-3-(5-methyl-2-p-totyl-oxazol-4-yl-methoxy)-cyclohexyloxymethyl]-benzoesäure-tert.-butylester werden in n-Heptan gelöst. 300 g (2,6 mol) Trifluoressigsäure werden zugegeben. Die Reaktion wird 24 Stunden bei RT gerührt und anschließend vollständig eingedampft. Der Rückstand wird in 1,0 L Diisopropylether aufgenommen und viermal mit je 500 mL Wasser gewaschen. Anschließend wird 1,0 L Wasser zugegeben und durch Zugabe von Natronlauge (4 M) ein basischer pH-Wert eingestellt. Nach Phasentrennung wird die wässrige Phase zweimal mit je 400 mL Diisopropylether gewaschen. Nach Zugabe von 1,6 L Diisopropylether wird mit Salzsäure (2 M) angesäuert. Die organische Phase wird nach Phasentrennung viermal mit je 500 mL Wasser gewaschen und anschließend auf ca. ein drittel des Volumens eingeengt. 2-Methyl-6-[(1R,3S)-3-(5-methyl-2-p-tolyl-oxazol-4-yl-methoxy)-cyclohexyloxymethyl]-benzoesäure fällt als kristalliner Feststoff aus und wird abfiltriert. Nach Trocknung im Vakuum erhält man 2-Methyl-6-[(1 R,3S)-3-(5-methyl-2-p-tolyl-oxazol-4-yl-methoxy)-cyclohexyloxymethyl]-benzoesäure in 66% Ausbeute.
c: aus Methyl-6-[(1 R,3S)-3-(5-methyl-2-p-tolyl-oxazol-4-yl-methoxy)-cyclohexyloxymethyl]-benzoesäurebenzylester: 15 g (27,8 mmol) Methyl-6-[(1R,3S)-3-(5-methyl-2-p-tolyl-oxazol-4-yl-methoxy)-cyclohexyloxymethyl]-benzoesäurebenzylester wird in 300 mL THF gelöst. 700 mg Palladium auf Kohle (5%ig) wird zugegeben. Anschließend wird bei Normaldruck und RT hydriert bis kein Wasserstoff mehr aufgenommen wird. Das Reaktionsgemisch wird filtriert und die organische Phase anschließend im Vakuum vollständig eingeengt. Der Rückstand wird in 150 mL tert.-Butylmethylether, 150 mL Wasser und 150 mL gesättigter Natriumhydrogencarbonatlösung aufgenommen. Nach Phasentrennung wird die wässrige Phase mit 150 mL tert.-Butylmethylether gewaschen. Anschließend wird 150 mL Diisopropylether zugegeben und mit konzentrierter Salzsäure angesäuert. Nach Phasentrennung wird die organische Phase viermal mit je 100 mL Wasser gewaschen und dann im Vakuum vollständig eingeengt. Der Rückstand wird mit wenig Diisopropylether verrührt. Der Feststoff wird filtriert und im Vakuum getrocknet. Man erhält 2-Methyl-6-[(1 R,3S)-3-(5-methyl-2-p-tolyl-oxazol-4-ylmethoxy)-cyclohexyloxymethyl]-benzoesäure in 42% Ausbeute als kristallinen Feststoff.

Die nach den verschiedenen Varianten hergestellte 2-Methyl-6-[(1R,3S)-3-(5-methyl-2-p-tolyl-oxazol-4-ylmethoxy)-cyclohexyloxymethyl]-benzoesäure hat ein Molekulargewicht von 449,55 (C₂₇H₃₁NO₅); MS (ESI): 450,29 [M + H]⁺.

Die nachfolgenden Beispiele 20 bis 21 sowie Schema V zeigen die Synthese einer weiteren Verbindung der allgemeinen Formel (I) auf.

### Schema V

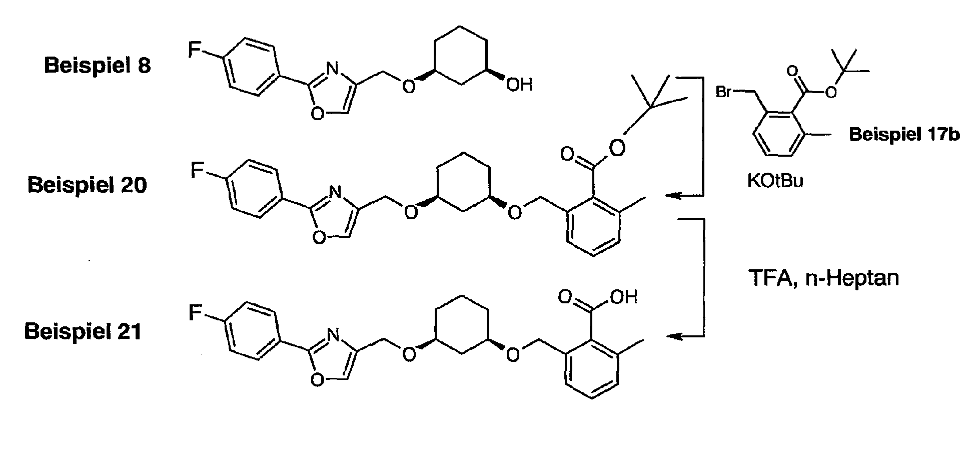

### Beispiel 20: Alkylierung von cis-3S-(2-(4-Fluorphenyl)-oxazol-4-yl-methoxy)-cyclohexan-1R-ol mit 2-Bromomethyl-6-methyl-benzoesäure-tert.-butylester zu cis-2-(3S-(2-(4-Fluorphenyl)-oxazol-4-yl-methoxy)-cyclo-hexyl-1R-oxymethyl)-6-methylbenzoesäure-tert.-butylester

Zu einer Lösung von 5,1 g (17,5 mmol) *cis*-3S-(2-(4-Fluorphenyl)-oxazol-4-yl-methoxy)-cyclohexan-1 R-ol (Beispiel 8) in 50 mL THF werden 3,31 g (28 mmol) Kalium-tert.-butylat gegeben. Das Gemisch wird 30 Minuten bei RT gerührt. 6,83 g (22,8 mmol) 2-Bromomethyl-6-methyl-benzoesäure-tert.-butylester (95%ig) (Beispiel 17b) werden zugegeben. Die Reaktion wird 1 Stunde bei RT gerührt. 100 mL Wasser und 17,5 mL Salzsäure (2 M) werden zugegeben. Nach Phasentrennung wird die organische Phase viermal mit je 100 mL Wasser gewaschen und im Vakuum vollständig eingeengt. Man erhält 10,6 g cis-2-(3S-(2-(4-Fluorphenyl)-oxazol-4-yl-methoxy)-cyclohexyl-1R-oxymethyl)-6-methylbenzoesäure-tert.-butylester als leicht gelbes Öl mit einer Reinheit von 82%. Der so erhaltene *cis*-2-(3S-(2-(4-Fluorphenyl)-oxazol-4-yl-methoxy)-cyclohexyl-1R-oxymethyl)-6-methylbenzoesäure-tert.-butylester hat ein Molekulargewicht von 495,59 (C₂₉H₃₄FNO₅); MS (ESI): 440,19 [M - tert.-Butyl + H + H]⁺.

### Beispiel 21: Spaltung des tert.-Butylesters von cis-2-(3S-(2-(4-Fluorphenyl)-oxazol-4-yl-methoxy)-cyclo-hexyl-1R-oxymethyl)-6-methylbenzoesäure-tert.-butylester zu cis-2-(3S-(2-(4-Fluorphenyl)-oxazol-4-yl-methoxy)-cyclohexyl-1R-oxymethyl)-6-methylbenzoesäure

10,6 g (17,5 mmol) cis-2-(3S-(2-(4-Fluorphenyl)-oxazol-4-yl-methoxy)-cyclohexyl-1R-oxymethyl)-6-methylbenzoesäure-tert.-butylester (82%ig) werden in 20 mL Dichlormethan gelöst. Nach Zugabe von 10 ml (128 mmol) Trifluoressigsäure wird die Reaktion 16 Stunden bei RT gerührt. Anschließend wird die Reaktion vollständig eingeengt. Der Rückstand wird in 100 mL Diisopropylether aufgenommen und sechsmal mit je 50 mL Wasser gewaschen. Anschließend werden 100 mL Wasser zugegeben und durch Zugabe von Natronlauge (2M) ein basischer pH-Wert eingestellt. Nach Phasentrennung wird die wässrige Phase dreimal mit je 50 mL Diisopropylether gewaschen. Dann werden 150 mL Diisopropylether zugegeben und mit Salzsäure (2M) angesäuert. Nach Phasentrennung wird die organische Phase dreimal mit je 50 mL Wasser gewaschen und dann vollständig eingeengt. Der Rückstand wird aus Diisopropylether umkristallisiert. Es werden 2,9 g cis-2-(3S-(2-(4-Fluorphenyl)-oxazol-4-yl-methoxy)-cyclohexyl-1 R-oxymethyl)-6-methylbenzoesäure als Kristalle isoliert. Die so hergestellte cis-2-(3S-(2-(4-Fluorphenyl)-oxazol-4-yl-methoxy)-cyclohexyl-1R-oxymethyl)-6-methylbenzoesäure hat ein Molekulargewicht von 439,48 (C₂₅H₂₆FNO₅): MS (ESI): 438,31 [M - H]⁻.

## Patentansprüche

1. **Verfahren zur Herstellung einer Verbindung der allgemeinen Formel (I) umfassend die Schritte gemäß dem folgenden Schema:** 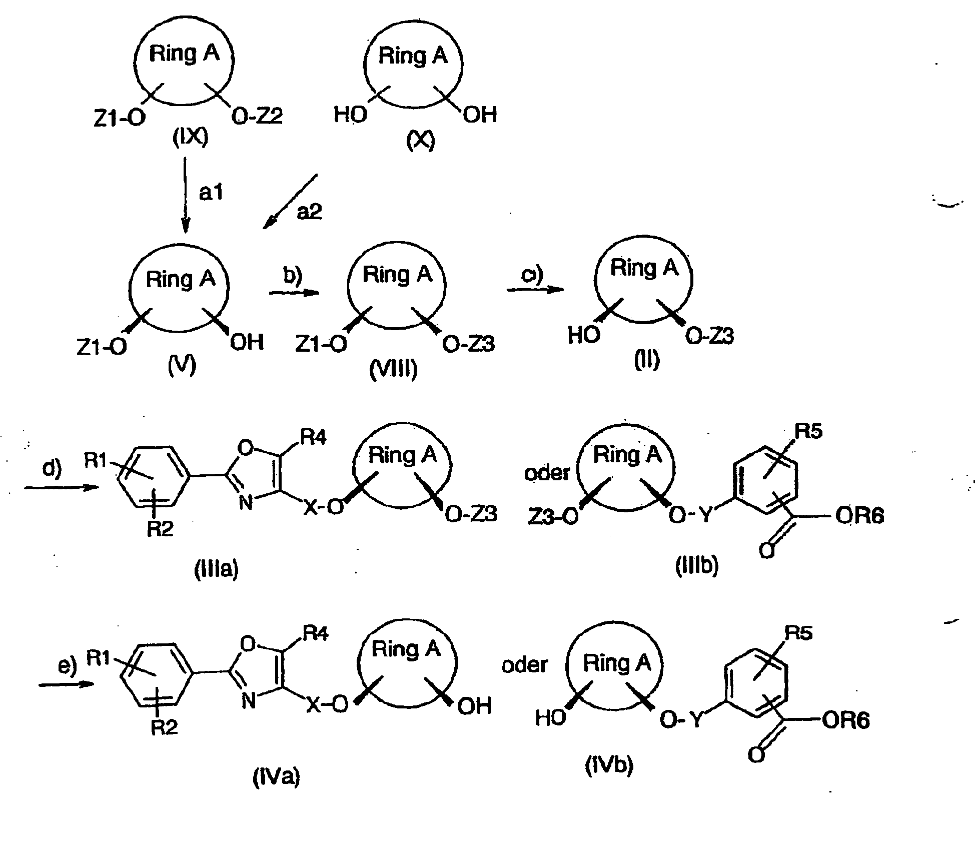 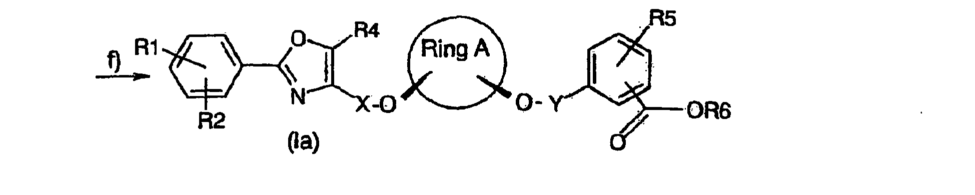 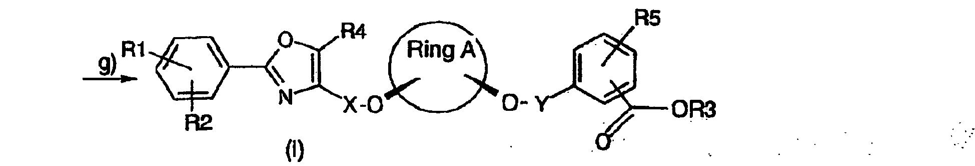 wobei in den einzelnen Schritten
a1) eine Verbindung der Formel (IX), mit Wasser zu einer Verbindung der Formel (V) in Gegenwart der Lipase B aus Candida antarctica umgesetzt wird, oder
a2) eine Verbindung der Formel (X) mit mindestens einem Acyldonor zur Verbindung (V) in Gegenwart der Lipase B aus Candida antarctica umgesetzt wird,
b) die Verbindung (V) in Gegenwart eines sauren Katalysators mit einer Verbindung, die die basenstabile und säurelabile Acetal -oder Ketal- Schutzgruppe Z3 ausbilden kann, zur Verbindung der Formel (VIII) umgesetzt wird und
c) die Verbindung (VIII) in Gegenwart eines Nukleophils zu einer Verbindung der Formel (II) umgesetzt wird,
d) die Verbindung (II) in Gegenwart einer Base B1 mit einer Verbindung der Formel (VI) zu einer Verbindung der Formel (IIIa) oder mit einer Verbindung der Formel (VII) zu einer Verbindung der Formel (IIIb) umgesetzt wird, 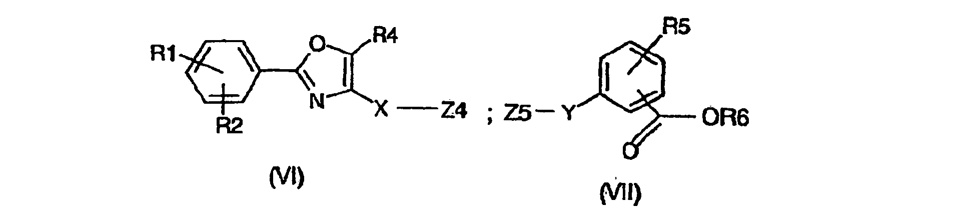
e) die Verbindung (IIIa) zu einer Verbindung der Formel (IVa) oder die Verbindung (IIIb) zu einer Verbindung der Formel (IVb) umgesetzt wird, wobei die jeweilige Umsetzung mit einem Alkohol in Gegenwart eines sauren Katalysators erfolgt,
f) die Verbindung (IVa) mit der Verbindung (VII) oder die Verbindung (IVb) mit der Verbindung (V1) zu einer Verbindung der Formel (Ia) in Gegenwart der Base B1 umgesetzt wird und
g) gegebenenfalls die Verbindung (la) zu der Verbindung (I) hydrolysiert oder hydrogenolysiert wird, sofern R3 gleich H ist,
wobei die Verbindungen (IX) und (X) jeweils als reines cis-isomer oder als cis/trans Gemische vorliegen,
und worin die Variablen und Substituenten jeweils die nachfolgende Bedeutung haben:
Ring A ist C₃-C₈-Cycloalkyl oder C₃-C₈-Cycloalkenyl, wobei in den Cycolalkyl-oder Cycloalkenylringen ein oder mehrere Kohlenstoffatome durch Sauerstoffatome ersetzt sein können,
R1, R2, R4 und R5 sind unabhängig voneinander H, F, Cl, Br, OH, NO₂, CF₃, OCF₃, C₁-C₆-Alkyl oder -O-(C₁-C₆-Alkyl),
R₃ ist H, C₁-C₆-Alkyl oder Benzyl, die gegebenenfalls mit F, Cl, Br, OH, NO₂, CF₃, OCF₃, C₁-C₈-Alkyl oder -O-(C₁-C₈-Alkyl) substituiert sein können,
R6 ist C₁-C₆-Alkyl oder Benzyl, die gegebenenfalls mit F,Cl, Br, OH, NO₂, CF₃, OCF₃, C₁-C₆-Alkyl oder -O-(C₁-C₆-Alkyl) substituiert sein können,
X ist C₁-C₆-Alkyl, wobei in der Alkylgruppe ein oder mehrere Kohlenstoffatome durch Sauerstoffatome ersetzt sein können,
Y ist C₁-C₆-Alkyl, wobei in der Alkylgruppe ein oder mehrere Kohlenstoffatome durch Sauerstoffatome ersetzt sein können,
Z1 und Z2 sind unabhängig voneinander eine säurestabile Schutzgruppe, aus der Reihe -C(O)-C₁-C₆ Alkyl oder C(O)- Phenyl,
Z3 ist eine basenstabile und säurelabile Acetal- oder Ketal- Schutzgruppe,
Z4 und Z5 sind unabhängig voneinander eine Abgangsgruppe,
B₁ ist ein tertiäres Erdalkalialkoholat, tertiäres Alkalialkoholat, Erdalkaliamid, Alkaliamid, Erdalkalisilazid, Alkalisilazid oder Alkalihydrid.

2. Verfahren gemäß Anspruch 1, **dadurch gekennzeichnet, dass** die Verbindung (IX) hergestellt wird durch
i) Umsetzung der Verbindung (X) mit mindestens einem Acyldonor in Gegenwart eines Enzyms, das hauptsächlich das cis-Isomere der Verbindung (IX) liefert, und die gegebenenfalls als Nebenprodukte entstehenden trans-Isomere der Verbindungen der Formel (V) abgetrennt werden, oder
ii) Umsetzung der Verbindung (X) mit mindestens einem Acyldonor.

3. Verfahren gemäß Anspruch 1 oder 2, **dadurch gekennzeichnet, dass** Z₁ und Z2 gleich -C(O)-CH₃ und/oder Z3 gleich Tetrahydropyranyl oder Methoxyisopropyl sind.

4. Verfahren gemäß einem der Ansprüche 1 bis 3, worin:
Ring A ist Cyclopentyl, Cyclohexyl oder Cycloheptyl,
R1, R2, R4 und R5 sind unabhängig voneinander H, F, Cl, Br, OH, NO₂, CF₃, - OCF₃, C₁-C₆-Alkyl oder O-C₁-C₆-Alkyl,
R3 ist H oder C₁-C₆-Alkyl oder Benzyl
X und Y sind unabhängig voneinander C₁-C₆-Alkyl.

5. Verfahren gemäß Anspruch 4, worin:
Ring A ist Cyclohexyl, wobei der X-enthaltende und der Y-enthaltende Substituent von Formel (I) in cis-1,3-Position zum Cyclohexyl-Fragment stehen,
X und Y sind Methyl.

6. Verfahren gemäß einem der Ansprüche 1 bis 4, worin
a) eine Verbindung der Formel (IX) in Gegenwart der Lipase B aus Candida antarctica mit Wasser zu einer Verbindung der Formel (V) umgesetzt wird,
b) die Verbindung (V) in Gegenwart eines sauren Katalysators mit einer Verbindung, die die basenstabile und saurelabile Acetal - oder Ketal - Schutzgruppe Z3 ausbilden kann, zur Verbindung der Formel (VIII) umgesetzt wird und
c) die Verbindung (VIII) in Gegenwart eines Nukleophils zur Verbindung der Formel (II) umgesetzt wird,
d) eine Verbindung (II) in Gegenwart einer Base B1 mit einer Verbindung der Formel (VI) zu einer Verbindung der Formel (IIIa) umgesetzt wird,
e) die Verbindung (IIIa) zu einer Verbindung der Formel (IVa) umgesetzt wird, wobei die Umsetzung mit einem Alkohol in Gegenwart eines sauren Katalysators erfolgt,
f) die Verbindung (IVa) mit der Verbindung (VII) zu einer Verbindung der Formel (Ia) in Gegenwart der Base B1 umgesetzt wird und
g) gegebenenfalls die Verbindung (la) zu der Verbindung (I) hydrolysiert oder hydrogenolysiert wird, sofern R3 gleich H ist, und
Ring A ist Cyclohexyl, wobei der X-enthaltende und der Y-enthaltende Substituent von Formel (I) in cis-1,3-Position zum Cyclohexyl-Fragment stehen und das Kohlenstoffatom des Ringes A, das mit dem Y-enthaltenden Substituenten substituiert ist, R-Konfiguration aufweist

7. Verfahren gemäß einem der Ansprüche 1 bis 6, **dadurch gekennzeichnet, dass** die Verbindung (I) in einer Enantiomerenreinheit von gröBer als 99 % vorliegt.

8. Verbindung der allgemeinen Formel (IIIa) 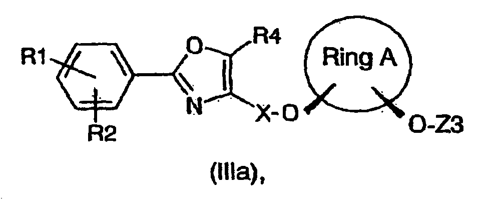 worin bedeuten:
R1, R2, R4 sind unabhängig voneinander H, F, Cl, Br, OH, NO₂, CF₃, OCF₃, C₁-C₆-Alkyl oder -O-(C₁-C₆-Alkyl),
X ist C₁-C₆-Alkyl, wobei in der Alkylgruppe ein oder mehrere Kohlenstoffatome durch Sauerstoffatome ersetzt sein können,
Ring A ist C₃-C₈-Cycloalkyl oder C₃-C₈-Cycloalkenyl, wobei in den Cycolalkyl- oder Cycloalkenylringen ein oder mehrere Kohlenstoffatome durch Sauerstoffatome ersetzt sein können,
Z3 ist eine basenstabile und säurelabile Acetal - oder Ketal - Schutzgruppe.

9. Verbindung gemäß Anspruch 8, worin in der Formel (IIIa) bedeuten:
Ring A ist Cyclohexyl, wobei der X-enthaltende und der Z3-enthaltende Substituent in cis-1,3-Position zum Cyclohexyl-Fragment stehen,
R1, R2 und R4 sind unabhängig voneinander H, F, Cl, C₁-C₃-Alkyl oder -O-(C₁-C₃-Alkyl),
Z3 ist Tetrahydropyranyl,
X ist Methyl.

10. Verbindung der allgemeinen Formel (IIIb) 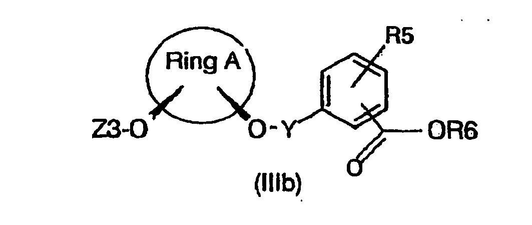 worin bedeuten:
R₅ ist unabhängig voneinander H, F, Cl, Br, OH, NO₂, CF₃, OCF₃, C₁-C₆-Alkyl oder -O-(C₁-C₆-Alkyl),
R6 ist C₁-C₆-Alkyl oder Benzyl, die gegebenenfalls mit F, Cl, Br, OH, NO₂, CF₃, OCF₃, C₁-C₆-Alkyl oder -O-(C₁-C₆-Alkyl substituiert sein können,
Y ist C₁-C₆-Alkyl, wobei in der Alkylgruppe ein oder mehrere Kohlenstoffatome durch Sauerstoffatome ersetzt sein können,
Ring A ist C₃₋C₈-Cycloalkyl oder C₃-C₈-Cycloalkenyl, wobei in den Cycolalkyl-oder Cycloalkenylringen ein oder mehrere Köhlenstoffatome durch Sauerstoffatome ersetzt sein können,
Z3 ist eine basenstabile und saurelabile Acetal-oder Ketal-Schutzgruppe.

11. Verbindung der allgemeinen Formel (VIII) 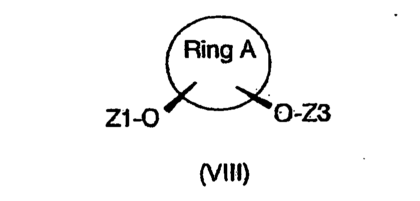 worin bedeuten:
Ring A ist Cyclohexyl, wobei der Z1-enthaltende und der Z3-enthaltende Substituent in cis-1,3-Position zum Cyclohexyl-Fragment stehen,
Z1 ist -C(O)CH₃,
Z3 ist Tetrahydropyranyl.

## Claims

1. A process for preparing a compound of the general formula (I), comprising the steps according to the following scheme: 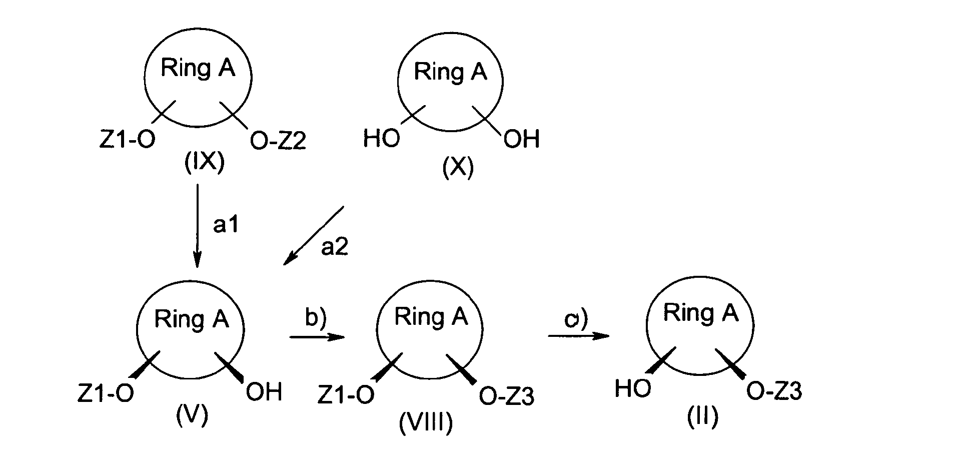 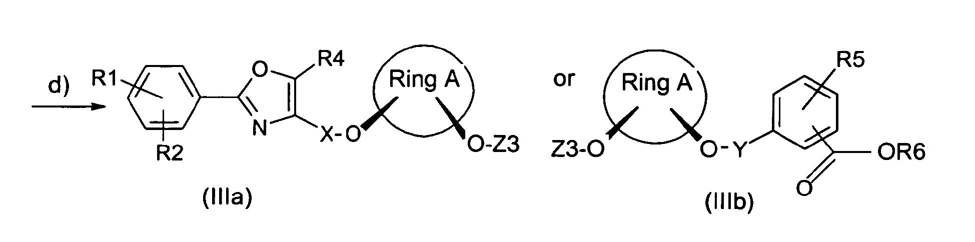 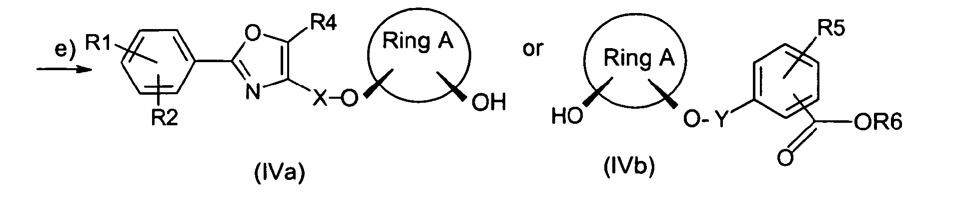 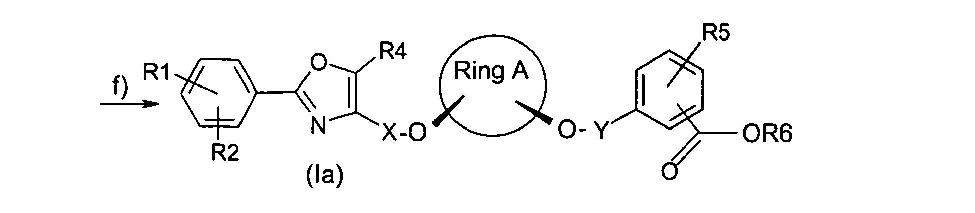 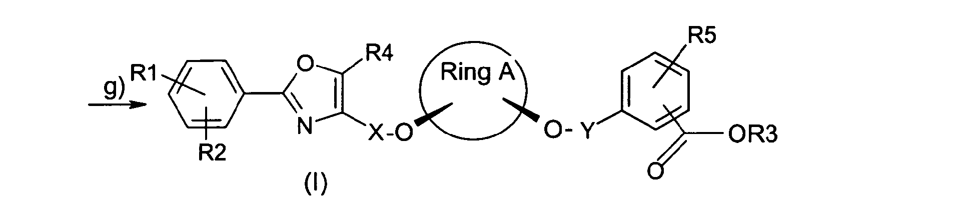 in which, in the individual steps,
a1) a compound of the formula (IX) is reacted with water to give a compound of the formula (V) in the presence of lipase B from Candida antartica, or
a2) a compound of the formula (X) is reacted with at least one acyl donor to give the compound (V) in the presence of lipase B from Candida antartica,
b) the compound (V) is reacted in the presence of an acidic catalyst with a compound which can form the base-stable and acid-labile acetal or ketal protecting group Z3 to give the compound of the formula (VIII) and
c) the compound (VIII) is converted in the presence of a nucleophile to a compound of the formula (II),
d) the compound (II) is reacted in the presence of a base B1 with a compound of the formula (VI) to give a compound of the formula (IIIa) or with a compound of the formula (VII) to give a compound of the formula (IIIb), 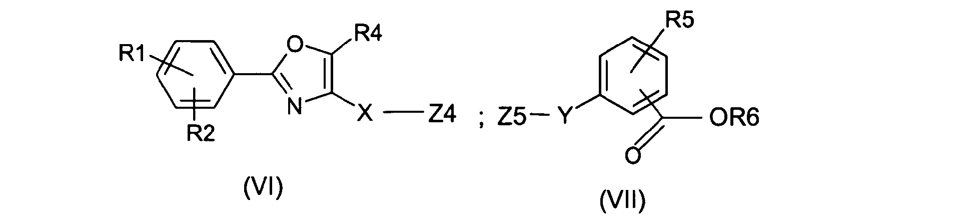
e) the compound (IIIa) is converted to a compound of the formula (IVa) or the compound (IIIb) to a compound of the formula (IVb), the particular reaction being effected with an alcohol in the presence of an acidic catalyst,
f) the compound (IVa) is reacted with the compound (VII) or the compound (IVb) with the compound (VI) to give a compound of the formula (Ia) in the presence of the base B1 and
g) if appropriate, the compound (Ia) is hydrolyzed or hydrogenolyzed to give the compound (I) when R3 is H,
the compounds (IX) and (X) each being present as the pure cis isomer or as cis/trans mixtures,
and in which the variables and substituents are each defined as follows:
Ring A is C₃-C₈-cycloalkyl or C₃-C₈-cycloalkenyl, in which one or more carbon atoms in the cycloalkyl or cycloalkenyl rings may be replaced by oxygen atoms,
R1, R2, R4 and R5 are each independently H, F, Cl, Br, OH, NO₂, CF₃, OCF₃, C₁₋C₆-alkyl or -O-(C₁-C₆-alkyl),
R3 is H, C₁-C₆-alkyl or benzyl, which may optionally be substituted by F, Cl, Br, OH, NO₂, CF₃, OCF₃, C₁-C₆-alkyl or -O-(C₁-C₆-alkyl),
R6 is C₁-C₆-alkyl or benzyl, which may optionally be substituted by F, Cl, Br, OH, NO₂ CF₃, OCF₃, C₁-C₆-alkyl or -O-(C₁-C₆-alkyl),
X is C₁-C₆-alkyl, in which one or more carbon atoms in the alkyl group may be replaced by oxygen atoms,
Y is C₁-C₆-alkyl, in which one or more carbon atoms in the alkyl group may be replaced by oxygen atoms,
Z1 and Z2 are each independently an acid-stable protecting group from the group consisting of -C(O)-C₁-C₆-alkyl or -C(O)-phenyl,
Z3 is a base-stable and acid-labile acetal or ketal protecting group,
Z4 and Z5 are each independently a leaving group,
B1 is a tertiary alkaline earth metal alkoxide, tertiary alkali metal alkoxide, alkaline earth metal amide, alkali metal amide, alkaline earth metal silazide, alkali metal silazide or alkali metal hydride.

2. The process as claimed in claim 1, wherein the compound (IX) is prepared by
i) reacting the compound (X) with at least one acyl donor in the presence of an enzyme which affords mainly the cis isomer of the compound (IX), and the trans isomers of the compounds of the formula (V) which may be formed as by-products are removed, or
ii) reacting the compound (X) with at least one acyl donor.

3. The process as claimed in claim 1 or 2, wherein Z1 and Z2 are each -C(O)-CH₃ and/or Z3 is tetrahydropyranyl or methoxyisopropyl.

4. The process as claimed in one of claims 1 to 3, in
which:
Ring A is cyclopentyl, cyclohexyl or cycloheptyl,
R1, R2, R4 and R5 are each independently H, F, Cl, Br, OH, NO₂, CF₃, -OCF₃, C₁-C₆-alkyl or O-C₁-C₆-alkyl,
R3 is H or C₁-C₆-alkyl or benzyl
X and Y are each independently C₁-C₆-alkyl.

5. The process as claimed in claim 4, in which:
Ring A is cyclohexyl in which the X-containing and the Y-containing substituents of formula (I) are in the cis-1,3-arrangement relative to the cyclohexyl fragment,
X and Y are each methyl.

6. The process as claimed in one of claims 1 to 4, in which
a) a compound of the formula (IX) is reacted in the presence of lipase B from Candida antarctica with water to give a compound of the formula (V),
b) the compound (V) is reacted in the presence of an acidic catalyst with a compound which can form the base-stable and acid-labile acetal or ketal protecting group Z3 to give the compound of the formula (VIII) and
c) the compound (VIII) is converted in the presence of a nucleophile to the compound of the formula (II),
d) a compound (II) is reacted in the presence of a base B1 with a compound of the formula (VI) to give a compound of the formula (IIIa),
e) the compound (IIIa) is converted to a compound of the formula (IVa), the reaction being effected with an alcohol in the presence of an acidic catalyst,
f) the compound (IVa) is reacted with the compound (VII) to give a compound of the formula (Ia) in the presence of the base B1 and
g) if appropriate, the compound (Ia) is hydrolyzed or hydrogenolyzed to give the compound (I) when R3 is H, and
Ring A is cyclohexyl in which the X-containing and
the Y-containing substituents of formula (I) are in the cis-1,3-arrangement relative to the cyclohexyl fragment, and the carbon atom of the ring A which is substituted by the Y-containing substituent has R configuration.

7. The process as claimed in one of claims 1 to 6, wherein the compound (I) is present in an enantiomeric purity of greater than 99%.

8. A compound of the general formula (IIIa) 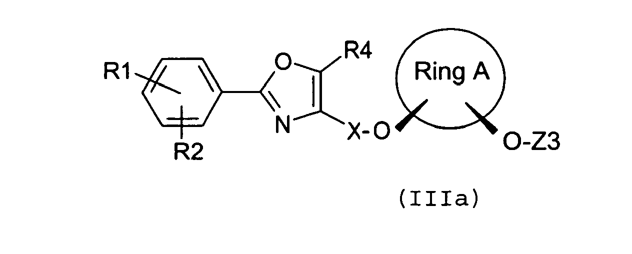 in which:
R1, R2, R4 are each independently H, F, Cl, Br, OH, NO₂, CF₃, OCF₃, C₁-C₆-alkyl or -O-(C₁-C₆-alkyl),
X is C₁-C₆-alkyl in which one or more carbon atoms in the alkyl group may be replaced by oxygen atoms,
Ring A is C₃-C₈-cycloalkyl or C₃-C₈-cycloalkenyl, in which one or more carbon atoms in the cycloalkyl or cycloalkenyl rings may be replaced by oxygen atoms,
Z3 is a base-stable and acid-labile acetal or ketal protecting group.

9. A compound as claimed in claim 8, in which, in the formula (IIIa) :
Ring A is cyclohexyl in which the X-containing and the Z3-containing substituents are in the cis-1,3-arrangement relative to the cyclohexyl fragment,
R1, R2 and R4 are each independently H, F, Cl, C₁-C₃-alkyl or -O-(C₁-C₃-alkyl)
Z3 is tetrahydropyranyl,
X is methyl.

10. A compound of the general formula (IIIb) 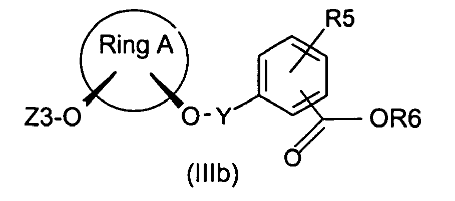 in which:
R5 is independently H, F, Cl, Br, OH, NO₂, CF₃, OCF₃, C₁-C₆-alkyl or -O-(C₁-C₆-alkyl),
R6 is C₁-C₆-alkyl or benzyl, which may optionally be substituted by F, Cl, Br, OH, NO₂, CF₃, OCF₃, C₁-C₆-alkyl or -O-(C₁-C₆-alkyl),
Y is C₁-C₆-alkyl in which one or more carbon atoms in the alkyl group may be replaced by oxygen atoms,
Ring A is C₃-C₈-cycloalkyl or C₃-C₈-cycloalkenyl, in which one or more carbon atoms in the cycloalkyl or cycloalkenyl rings may be replaced by oxygen atoms,
Z3 is a base-stable and acid-labile acetal or ketal protecting group.

11. A compound of the general formula (VIII) 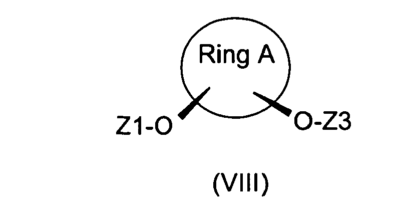 in which:
Ring A is cyclohexyl in which the Z1-containing and the Z3-containing substituents are in the cis-1,3-arrangement relative to the cyclohexyl fragment,
Z1 is -C(O)CH₃,
Z3 is tetrahydropyranyl.

## Revendications

1. Procédé de préparation d'un composé de formule générale (I), comprenant les étapes selon le schéma suivant : 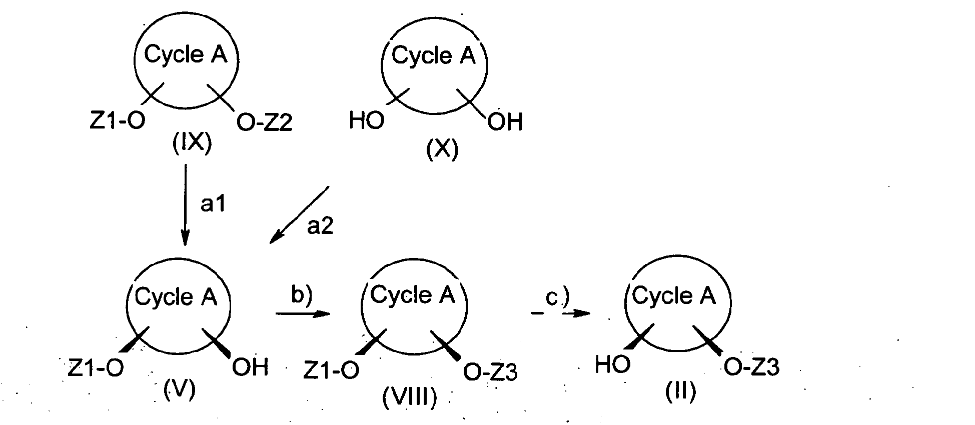 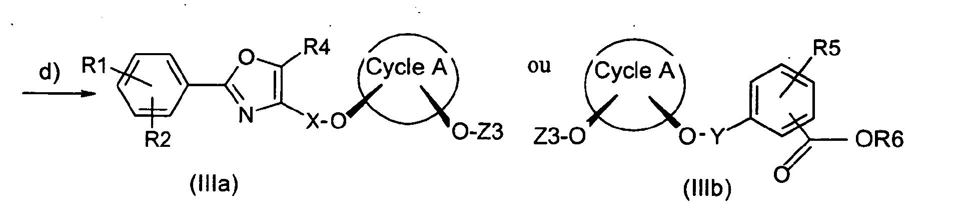 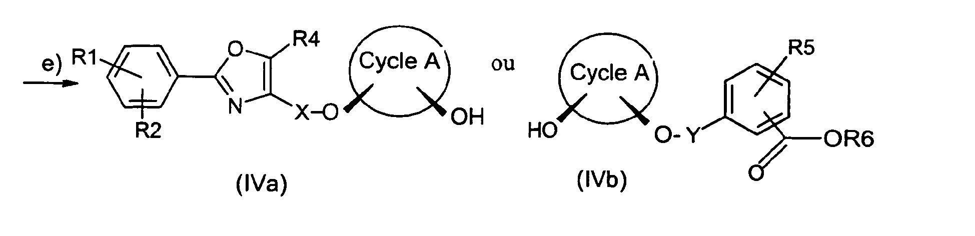 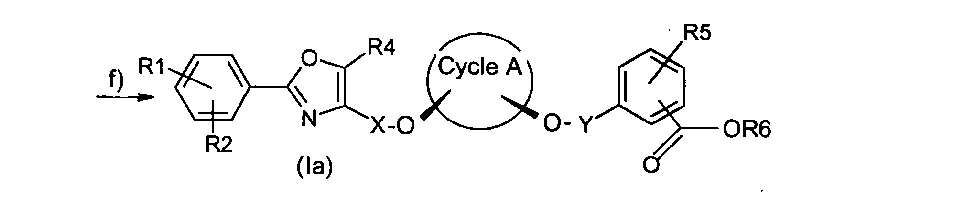 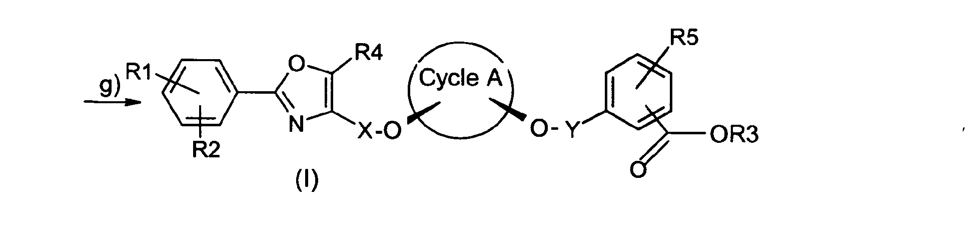 dans lequel, dans les étapes individuelles,
a1) un composé de formule (IX) est mis en réaction avec de l'eau pour donner un composé de formule (V) en présence de la lipase B de Candida antarctica, ou
a2) un composé de formule (X) est mis en réaction avec au moins un donneur d'acyle pour donner le composé (V) en présence de la lipase B de Candida antarctica,
b) le composé (V) est mis en réaction en présence d'un catalyseur acide avec un composé qui peut former le groupement protecteur d'acétal ou de cétal Z3 stable aux bases et labile aux acides pour donner le composé de formule (VIII) et
c) le composé (VIII) est transformé en présence d'un nucléophile en un composé de formule (II),
d) le composé (II) est mis en réaction en présence d'une base B1 avec un composé de formule (VI) pour donner un composé de formule (IIIa) ou avec un composé de formule (VII) pour donner un composé de formule (IIIb), 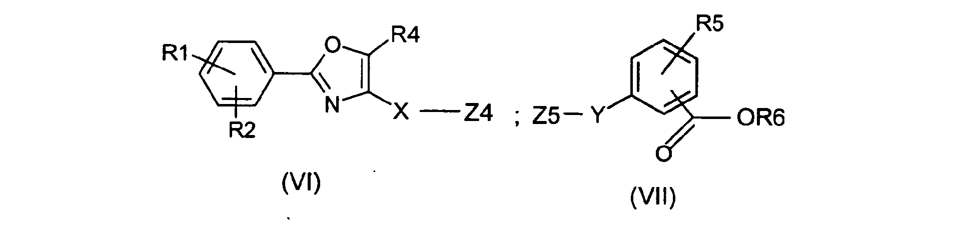
e) le composé (IIIa) est transformé en un composé de formule (IVa) ou le composé (IIIb) en un composé de formule (IVb), la réaction particulière étant effectuée avec un alcool en présence d'un catalyseur acide,
f) le composé (IVa) est mis en réaction avec le composé (VII) ou le composé (IVb) avec le composé (VI) pour donner un composé de formule (la) en présence de la base B1 et
g) le cas échéant, le composé (la) est hydrolysé ou hydrogénolysé en le composé (I) lorsque R3 est H,
les composés (IX) et (X) étant chacun présents sous forme de l'isomère cis pur ou sous forme de mélanges cis/trans,
et dans lequel les variables et les substituants sont chacun définis comme suit :
Le cycle A est C₃-C₈-cycloalkyle ou C₃-C₈-cycloalcényle, dans lesquels un ou plusieurs atomes de carbone dans les cycles cycloalkyle ou cycloalcényle peuvent être remplacés par des atomes d'oxygène,
R1, R2, R4 et R5 sont chacun indépendamment H, F, Cl, Br, OH, NO₂, CF₃, OCF₃, C₁-C₆-alkyle ou -O-(C₁-C₆-alkyle),
R3 est H, C₁-C₆-alkyle ou benzyle, qui peuvent éventuellement être substitués par F, Cl, Br, OH, NO₂, CF₃, OCF₃, C₁-C₆-alkyle ou -O-(C₁-C₆-alkyle),
R6 est C₁-C₆-alkyle ou benzyle, qui peuvent éventuellement être substitués par F, Cl, Br, OH, NO₂, CF₃, OCF₃, C₁-C₆-alkyle ou -O-(C₁-C₆-alkyle),
X est C₁-C₆-alkyle, dans lequel un ou plusieurs atomes de carbone dans le groupement alkyle peuvent être remplacés par des atomes d'oxygène,
Y est C₁-C₆-alkyle, dans lequel un ou plusieurs atomes de carbone dans le groupement alkyle peuvent être remplacés par des atomes d'oxygène,
Z1 et Z2 sont chacun indépendamment un groupement protecteur stable aux acides choisi parmi -C(O)-C₁-C₆-alkyle ou -C(O)-phènyle,
Z3 est un groupement protecteur d'acétal ou de cétal stable aux bases et labile aux acides,
Z4 et Z5 sont chacun indépendamment un groupement partant,
B1 est un alcoxyde de métal alcalino-terreux tertiaire, un alcoxyde de métal alcalin tertiaire, un amide de métal alcalino-terreux, un amide de métal alcalin, un silazide de métal alcalino-terreux, un silazide de métal alcalin ou une hydrure de métal alcalin.

2. Procédé selon la revendication 1, **caractérisé en ce que** le composé (IX) est préparé par
i) la réaction du composé (X) avec au moins un donneur d'acyle en présence d'une enzyme qui produit principalement l'isomère cis du composé (IX), et les isomères trans des composés de formule (V) qui peuvent être formés en tant que sous-produits sont éliminés, ou
ii) la réaction du composé (X) avec au moins un donneur d'acyle.

3. Procédé selon la revendication 1 ou 2, **caractérisé en ce que** Z1 et Z2 sont chacun -C(O)-CH₃ et/ou Z3 est tétrahydropyranyle ou méthoxyisopropyle.

4. Procédé selon l'une des revendications 1 à 3, dans lequel :
Le cycle A est cyclopentyle, cyclohexyle ou cycloheptyle,
R1, R2, R4 et R5 sont chacun indépendamment H, F, Cl, Br, OH, NO₂, CF₃, -OCF₃, C₁-C₆-alkyle ou O-C₁-C₆-alkyle,
R3 est H ou C₁-C₆-alkyle ou benzyle
X et Y sont chacun indépendamment C₁-C₆-alkyle.

5. Procédé selon la revendication 4, dans lequel :
Le cycle A est cyclohexyle dans lequel les substituants contenant X et contenant Y de formule (I) sont dans l'arrangement cis-1,3 par rapport au fragment cyclohexyle,
X et Y sont chacun méthyle.

6. Procédé selon l'une des revendications 1 à 4, dans lequel
a) un composé de formule (IX) est mis en réaction en présence de la lipase B de Candida antarctica avec de l'eau pour donner un composé de formule (V),
b) le composé (V) est mis en réaction en présence d'un catalyseur acide avec un composé qui peut former le groupement protecteur d'acétal ou de cétal Z3 stable aux bases et labile aux acides pour donner le composé de formule (VIII) et
c) le composé (VIII) est transformé en présence d'un nucléophile en le composé de formule (II),
d) un composé (II) est mis en réaction en présence d'une base B1 avec un composé de formule (VI) pour donner un composé de formule (IIIa),
e) le composé (IIIa) est transformé en un composé de formule (IVa), la réaction étant effectuée avec un alcool en présence d'un catalyseur acide,
f) le composé (IVa) est mis en réaction avec le composé (VII) pour donner un composé de formule (la) en présence de la base B1 et
g) le cas échéant, le composé (la) est hydrolysé ou hydrogénolysé en composé (I) lorsque R3 est H, et
le cycle A est cyclohexyle dans lequel les substituants contenant X et contenant Y de la formule (I) sont dans l'arrangement cis-1,3 par rapport au fragment cyclohexyle, et l'atome de carbone du cycle A qui est substitué par le substituant contenant Y a la configuration R.

7. Procédé selon l'une des revendications 1 à 6, **caractérisé en ce que** le composé (I) se présente dans une pureté énantiomérique supérieure à 99 %.

8. Composé de formule générale (IIIa) 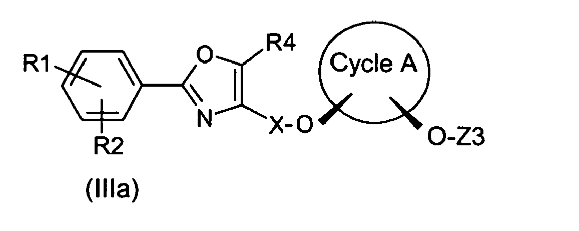 dans laquelle :
R1, R2, R4 sont chacun indépendamment H, F, Cl, Br, OH, NO₂, CF₃, OCF₃, C₁-C₆-alkyle ou -O-(C₁-C₆-alkyle),
X est C₁-C₆-alkyle dans lequel un ou plusieurs atomes de carbone dans le groupement alkyle peuvent être remplacés par des atomes d'oxygène,
le cycle A est C₃-C₈-cycloalkyle ou C₃-C₈-cycloalcényle, dans lesquels un ou plusieurs atomes de carbone dans les cycles cycloalkyle ou cycloalcényle peuvent être remplacés par des atomes d'oxygène,
Z3 est un groupement protecteur d'acétal ou de cétal stable aux bases et labile aux acides.

9. Composé selon la revendication 8, dans lequel, dans la formule (IIIa) :
le cycle A est cyclohexyle dans lequel les substituants contenant X et contenant Z3 sont dans l'arrangement cis-1,3 par rapport au fragment cyclohexyle,
R1, R2 et R4 sont chacun indépendamment H, F, Cl, C₁-C₃-alkyle ou -O-(C₁-C₃-alkyle),
Z3 est tétrahydropyranyle,
X est méthyle.

10. Composé de formule générale (IIIb) 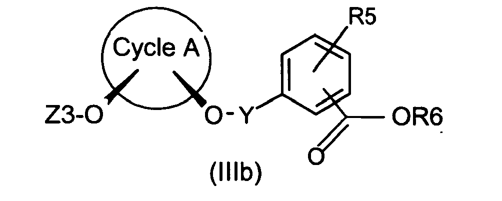 dans laquelle :
R5 sont chacun indépendamment H, F, Cl, Br, OH, NO₂, CF₃, OCF₃, C₁-C₆-alkyle ou -O-(C₁₋C₆-alkyle),
R6 est C₁-C₆-alkyle ou benzyle, qui peuvent éventuellement être substitués par F, Cl, Br, OH, NO₂, CF₃, OCF₃, C₁-C₆-alkyle ou -O-(C₁-C₆-alkyle),
Y est C₁-C₆-alkyle dans lequel un ou plusieurs atomes de carbone dans le groupement alkyle peuvent être remplacés par des atomes d'oxygène,
le cycle A est C₃-C₈-cycloalkyle ou C₃-C₈-cycloalcényle, dans lesquels un ou plusieurs atomes de carbone dans les cycles cycloalkyle ou cycloalcényle peuvent être remplacés par des atomes d'oxygène,
Z3 est un groupement protecteur d'acétal ou de cétal stable aux bases et labile aux acides.

11. Composé de formule générale (VIII) 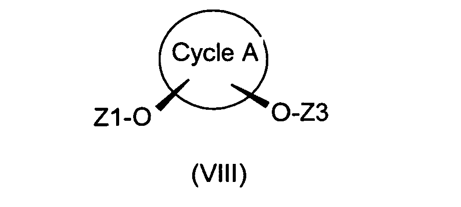 dans laquelle :
le cycle A est cyclohexyle dans lequel les substituants contenant Z1 et contenant Z3 sont dans l'arrangement cis-1,3 par rapport au fragment cyclohexyle,
Z1 est -C(O)CH₃,
Z3 est tétrahydropyranyle.
